# EUROPEAN PATENT APPLICATION

(11) **EP 4 640 669 A1**
(43) Date of publication of application: **29.10.2025**
(21) Application number: 23907909.8
(22) Date of filing: 14.12.2023
(51) Int. Cl.: C07C 237/24, C07C 219/12, A61K 9/51, A61K 47/18, A61K 47/14, A61K 48/00

(54) **CYCLOALKANE-BASED LIPID COMPOUND FOR DELIVERING NUCLEIC ACIDS, AND LIPID NANOPARTICLES COMPRISING SAME**

(30) Priority: 19.12.2022 KR 20220177848
(71) Applicant: GREEN CROSS CORPORATION, Yongin-si, Gyeonggi-do 16924 (KR)
(72) Inventor: DONG, Yizhou, New York 10128 (US); ZHANG, Yuebao, Ziyang, Sichuan 642350 (CN); KANG, Diana Daeun, Columbus, Ohio 43220 (US); DU, Shi, Columbus, Ohio 43202 (US)
(74) Representative: Dompatent Partnerschaft von Patent- und Rechtsanwälten mbB
(86) International application number: PCT/KR2023/095111
(87) International publication number: WO 2024/136626

(57) **Abstract**

The present invention provides a cycloalkane-based lipid compound of Formula 1 and a pharmaceutically acceptable salt thereof. The cycloalkane-based lipid compound is a compound having a form in which carbonyl-based substituents are bonded to the central structure of a cycloalkane. The cycloalkane-based lipid compound according to the present invention is used as an ionizable lipid, which is a component of a lipid nanoparticle for delivering a nucleic acid.

## Description

### [Technical Field]

The present invention relates to a cycloalkane-based lipid compound for delivering a nucleic acid and a lipid nanoparticle comprising the same.

This application claims the benefit of priority based on Korean Patent Application No. 10-2022-0177848 filed on December 19, 2022, the entire contents of which are incorporated herein as part of the present specification.

### [Background Art]

In drug delivery, drug delivery system (DDS) aims to deliver the required amount of drug to target site and reduce the side effects of the drug, in order to maximize its efficacy and effectiveness. The drug delivery system is a high-value technology to generate economic benefits comparable to development of new drugs and may contribute to improving the quality of patient treatment.

In particular, several studies related to the development of delivery system for nucleic acids have been conducted to control the desired responses with biological drugs. Nucleic acids such as antisense RNA, siRNA and mRNA are substances that can inhibit the expression of specific proteins *in vivo,* and are attracting attention as important tools in the treatment of cancer, hereditary diseases, infectious diseases, autoimmune diseases, and the like. Some nucleic acids, for example, mRNA or plasmids lead to the expression of specific cellular products useful, for example, in the treatment of diseases associated with deficiencies of proteins or enzymes. Delivery of nucleic acids increases the levels of existing proteins, thereby replace deficient or non-functional versions of proteins, and also introduce new proteins and related functionality into a cell or organism. Nucleic acid-based therapeutic agents have tremendous potential, but to realize this potential, there is a need to more effectively deliver nucleic acids to the appropriate site within a cell or organism.

In delivering nucleic acids containing genetic information, viral carriers may be effective, but the use of viral carriers is limited due to several drawbacks such as immunogenicity, limitations in the size of the injected DNA and difficulties in mass production, and thus, non-viral carriers such as cationic liposomes and polymers are attracting attention as alternatives thereof. However, these non-viral carriers may exhibit remarkably high cytotoxicity due to poor biocompatibility and non-biodegradability, and are limited by low transfection efficiency.

Accordingly, as a result of continuous research on a lipid nanoparticle as non-viral carriers, the present inventors have completed the invention by synthesizing a novel ionizable lipid compound suitable as a component of the lipid nanoparticle.

### [Prior Art Document]

### [Patent Document]

(Patent Document 1) US Patent No. 10166298

### [Disclosure]

### [Technical Problem]

It is an object of the present invention to provide a cycloalkane-based lipid compound that enables the prevention or treatment of related diseases by efficiently delivering a nucleic acid in a lipid nanoparticle to cells and animals, as an ionized lipid, which is a core component of the lipid nanoparticle for delivering the nucleic acid.

### [Technical Solution]

According to a first aspect of the present invention,
the present invention provides a cycloalkane-based lipid compound of Formula 1 below and a pharmaceutically acceptable salt thereof:

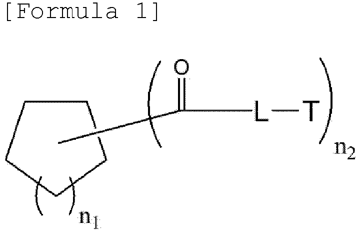

According to one embodiment of the present invention, n₁ is 1 to 4.

According to one embodiment of the present invention, n₂ is 1 to 4.

According to one embodiment of the present invention, when n₂ is 2 or 3, each of the substituents linked to the cycloalkane is independent.

According to one embodiment of the present invention, L is 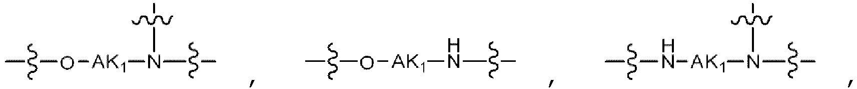 or

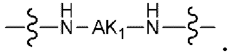

According to one embodiment of the present invention, AK₁ is alkylene having 1 to 5 carbon atoms.

According to one embodiment of the present invention, T is alkyl or alkenyl having 6 to 20 carbon atoms, or

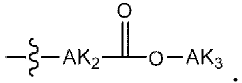

According to one embodiment of the present invention, AK₂ is alkylene having 6 to 10 carbon atoms.

According to one embodiment of the present invention, AK₃ is alkyl or alkenyl having 6 to 10 carbon atoms.

According to one embodiment of the present invention, when n₂ is 2 or 3, the substituents linked to the cycloalkane are spaced apart so that one or more carbon atoms are located therebetween.

According to one embodiment of the present invention, n₁ is 2.

According to one embodiment of the present invention, n₂ is 3.

According to one embodiment of the present invention, AK₁ is alkylene having 3 to 5 carbon atoms.

According to one embodiment of the present invention, T is alkyl having 8 to 16 carbon atoms.

According to one embodiment of the present invention, AK₂ is alkylene having 8 to 10 carbon atoms, and AK₃ is alkyl or alkenyl having 8 to 10 carbon atoms.

According to one embodiment of the present invention, the cycloalkane-based lipid compounds are Compounds 1 to 40.

According to a second aspect of the present invention,
the present invention provides a lipid nanoparticle comprising the cycloalkane-based lipid compound as described above, phospholipid, cholesterol and PEG-lipid conjugate.

According to one embodiment of the present invention, the lipid nanoparticle comprises 10 mol% to 40 mol% of the cycloalkane lipid compound based on the total number of moles of the cycloalkane-based lipid compound, phospholipid, cholesterol and PEG-lipid conjugate.

### [Advantageous Effects]

In the case of lipid nanoparticles used to deliver nucleic acids, the ionized lipid in the composition is known to have the greatest effect on efficacy. Specifically, it has a more direct effect on nucleic acid delivery efficiency than other compositions by enabling the escape of nucleic acids from endosomes within cells. Thus, in order to develop a nucleic acid-based gene therapeutic agent with increased efficacy, there is a need for a novel ionized lipid with increased delivery efficiency. The present inventors have synthesized a cycloalkane-based lipid compound and evaluated its nucleic acid delivery ability in cell and animal models, thereby confirming its usefulness as a lipid nanoparticle composition required for the development of a nucleic acid therapeutic agent.

### [Description of Drawings]

Figure 1 is a graph showing the results of particle size (Z-average and polydispersity index (PDI)) measured according to Experimental Example 1.
Figure 2 is a graph showing the results of luminescence intensity (normalized luminescence) measured according to Experimental Example 1.
Figure 3 is a graph showing the results of particle size (Z-average and polydispersity index (PDI)) measured for lipid nanoparticles for intramuscular injection according to Experimental Example 2.
Figure 4 is a graph showing the results of the luminescence intensity (total flux) measured for lipid nanoparticles for intramuscular injection according to Experimental Example 2.
Figure 5 is a graph showing the results of particle size (Z-average and polydispersity index (PDI)) measured for lipid nanoparticles for intravenous injection according to Experimental Example 2.
Figure 6 is a graph showing the results of the luminescence intensity (total flux) measured for lipid nanoparticles for intravenous injection according to Experimental Example 2.

### [Best Mode]

The embodiments provided according to the present invention can be all achieved by the following description. It should be understood that the following description describes preferred embodiments of the present invention and the present invention is not necessarily limited thereto.

The present invention provides a cycloalkane-based lipid compound and a lipid nanoparticle comprising the same, for delivering a nucleic acid. It is not limited to the cycloalkane lipid compound itself according to the present invention, but extends to isomers such as tautomers or stereoisomers, and includes pharmaceutically acceptable salts thereof.

The "pharmaceutically acceptable salt" includes both acid addition salts and base addition salts. The acid addition salt may be a salt with, for example, hydrochloric acid, trifluoroacetic acid, formic acid, citric acid, fumaric acid, monosodium fumarate, p-toluenesulfonic acid, stearic acid, disodium citrate, tartaric acid, malic acid, lactic acid, succinic acid, salicylic acid, or the like, but is not particularly limited as long as it is an acid addition salt commonly used in the relevant technical field. The base addition salt may be a salt with, for example, ammonium, sodium, potassium, calcium, magnesium, isopropyl amine, diethylamine, ethanolamine, trimethylamine, dicyclohexylamine, choline, caffeine, or the like, but is not particularly limited as long as it is a base addition salt commonly used in the relevant technical field.

The cycloalkane-based lipid compound according to the present invention may be an ionizable lipid whose charge state changes depending on the surrounding pH. These ionizable lipids have properties similar to lipids and may play a role in ensuring that the drug is encapsulated within lipid nanoparticles with high efficiency through electrostatic interaction with the drug (e.g., anionic drug and/or nucleic acid).

As used in the specification, the term "alkyl" refers to a straight or branched saturated hydrocarbon containing one radical, wherein the one radical is a functional group that determines the bonding position, and the bonding position is not particularly limited. Examples of the term "alkyl" include, but are not necessarily limited to, methyl, ethyl, n-propyl, **i-**propyl, n-butyl, i-butyl, t-butyl, n-pentyl, i-pentyl, hexyl, and the like.

As used in the specification, the term "alkylene" refers to a straight or branched saturated hydrocarbon containing two radicals, wherein each of the two radicals is a functional group that determines the bonding position, and the bonding position is not particularly limited. Examples of the term "alkylene" include, but are not necessarily limited to, methylene, ethylene, and the like.

As used in the specification, the term "alkenyl" refers to a straight or branched hydrocarbon containing one radical and having one or more carbon-carbon double bonds, wherein the one radical is a functional group that determines the bonding position, and the bonding position is not particularly limited. Examples of the term "alkenyl" include, but are not necessarily limited to, ethenyl, propenyl, and the like.

Hereinafter, specific examples of the cycloalkane-based lipid compound according to the present invention will be described in detail.

One embodiment of the present invention provides a cycloalkane-based lipid compound or a pharmaceutically acceptable salt thereof. In this case, the "pharmaceutically acceptable salt" may be an acid addition salt or a base addition salt according to the above description.

The cycloalkane-based lipid compound according to one embodiment of the present invention may be a compound having the structure shown in following Formula 1:

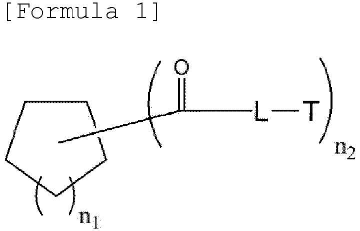

As can be confirmed in Formula 1 above, the ionizable lipid compound according to the present invention has a central structure of cycloalkane, and is therefore named "cycloalkane-based lipid compound".

In Formula 1 above, n₁ specifies the number of carbon atoms forming the cycloalkane ring. n₁ is defined as a natural number with no decimal places, and according to one embodiment of the present invention, the n₁ is 1 to 4. If n₁ is 1, the cycloalkane becomes cyclopentane in the form of a pentagonal ring, and if n₁ is 2, the cycloalkane becomes cyclohexane in the form of a hexagonal ring. In addition, if n₁ is 3, the cycloalkane becomes cycloheptane in the form of a heptagonal ring, and if n₁ is 4, the cycloalkane becomes cyclooctane in the form of an octagonal ring. When n₁ is 1 to 4, the bond angle with adjacent carbons is appropriate, and may therefore form a more stable ring structure. The cycloalkane-based lipid compound according to one embodiment of the present invention may be a compound of Formula 1 wherein n₁ is 1 to 4, 1 to 3, 1 to 2, or 2.

In Formula 1 above, n₂ specifies the number of carbonyl-based substituents linked to the cycloalkane. In the portion where the carbonyl-based substituent is not linked, there may be no substituent, or an alkyl group and the like having 1 to 4 carbon atoms, which may be generally introduced in the relevant technical field within the range where structural similarity is recognized, may be introduced. n₂ is defined as a natural number with no decimal places, and according to one embodiment of the present invention, the n₂ is 1 to 4. When n₂ is 2 to 4, a plurality of substituents are freely linked to the carbon atoms forming the cycloalkane ring, wherein each of the substituents is independently defined. According to one embodiment of the present invention, when n₂ is 2 to 4, the substituents linked to the cycloalkane are spaced apart so that one or more carbon atoms are located therebetween. When a plurality of substituents are positioned spaced apart from each other, steric hindrance between the substituents may be reduced, and functionality may be improved by a more dispersed arrangement of the substituents. The cycloalkane-based lipid compound according to one embodiment of the present invention may be a compound of Formula 1 wherein n₂ is 1 to 4, 2 to 4, 2 to 3, or 3.

The carbonyl-based substituents linked to the cycloalkane have a structure in which a linking group (L) and a terminal group (T) are linked to the carbonyl. According to one embodiment of the present invention, the linking group (L) is 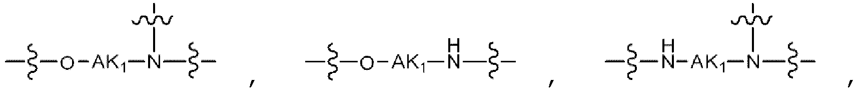 or 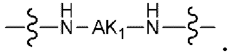 The linking group (L) is expressed in the same positional relationship as in Formula 1, and is linked to the carbonyl carbon at the left linking point and to the terminal group (T) at the right linking point. If the nitrogen on the right has two connection points, they are all linked to the terminal group (T). However, in this case, the two terminal groups (T) linked to the nitrogen on the right are defined independently. The cycloalkane-based lipid compound according to one embodiment of the present invention may be a compound of Formula 1 wherein the linking group (L) is 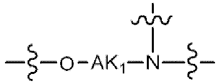 or

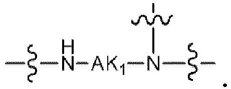

According to one embodiment of the present invention, AK₁ in the linking group (L) is alkylene having 1 to 5 carbon atoms. Since the binding position on AK₁ is not specified to the terminal carbon, a branched alkyl substituent may be formed when bonded to a non-terminal carbon. In the cycloalkane-based lipid compound according to one embodiment of the present invention, AK₁ in the linking group (L) may be alkylene having 2 to 5 or 3 to 5 carbon atoms.

In the carbonyl-based substituents, the terminal group (T) is linked to the right nitrogen on the linking group (L) to form one end of the compound. The terminal group (T) may be alkyl or alkenyl, or may be a form in which an ester group is introduced into the middle of the alkyl or alkenyl chain. According to one embodiment of the present invention, the terminal group (T) is alkyl or alkenyl having 6 to 20 carbon atoms, or 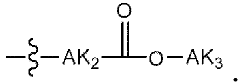 When the terminal group (T) is alkyl or alkenyl having 6 to 20 carbon atoms, since the binding position on the alkyl or alkenyl is not specified to the terminal carbon, a branched alkyl substituent may be formed when bonded to a non-terminal carbon. In the cycloalkane-based lipid compound according to one embodiment of the present invention, the terminal group (T) may be alkyl or alkenyl having 8 to 20, 8 to 18 or 8 to 16 carbon atoms.

According to one embodiment of the present invention, when the terminal group (T) is 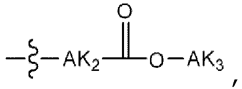 AK₂ may be alkylene having 6 to 10 or 8 to 10 carbon atoms, and AK₃ may be alkyl or alkenyl having 6 to 10 or 8 to 10 carbon atoms. Since the binding position on AK₂ or AK₃ is not specified to the terminal carbon, a branched alkyl substituent may be formed when bonded to a non-terminal carbon. In the cycloalkane-based lipid compound according to one embodiment of the present invention, in the terminal group (T), AK₂ may be alkylene having 8 carbon atoms, and AK₃ may be alkyl or alkenyl having 8 or 9 carbon atoms.

The cycloalkane-based lipid compound according to one embodiment of the present invention may be a compound in which three carbonyl-based substituents are linked to cyclohexane and one carbon is located between the substituents, and the carbonyl-based substituents are therefore uniformly dispersed. In addition, the three carbonyl-based substituents may have the same structure.

According to one embodiment of the present invention, the cycloalkane-based lipid compounds are Compounds 1 to 40 in Table 1 below.

**[Table 1]**

| Compound | Structural Formula |
|---|---|
| 1 | 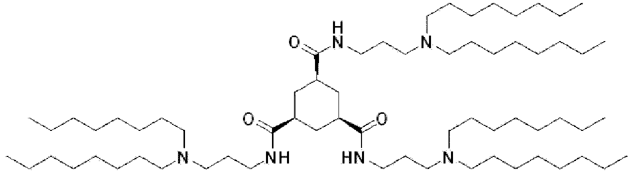 |
| 2 | 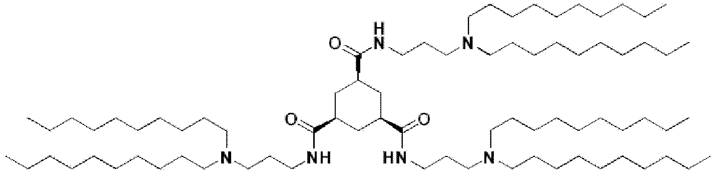 |
| 3 | 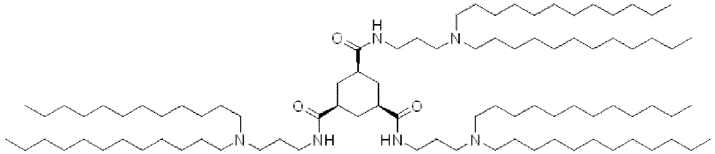 |
| 4 | 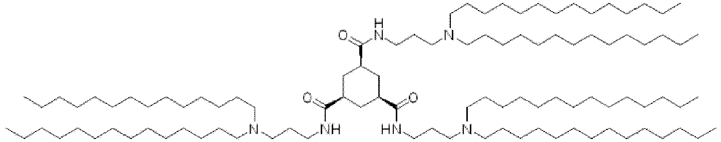 |
| 5 | 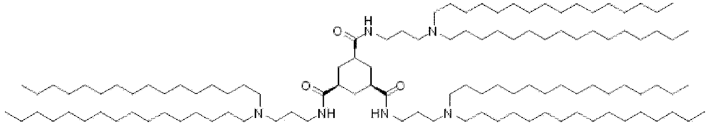 |
| 6 | 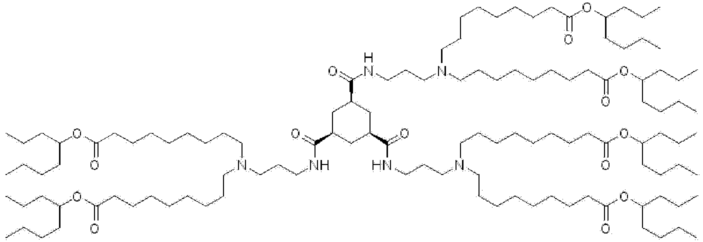 |
| 7 | 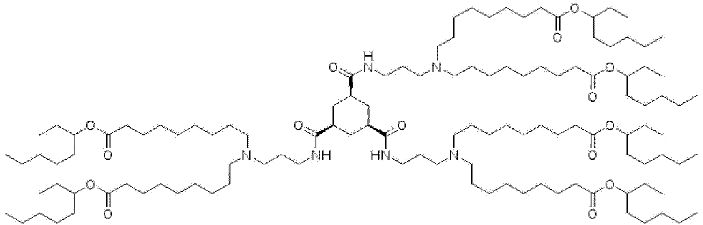 |
| 8 | 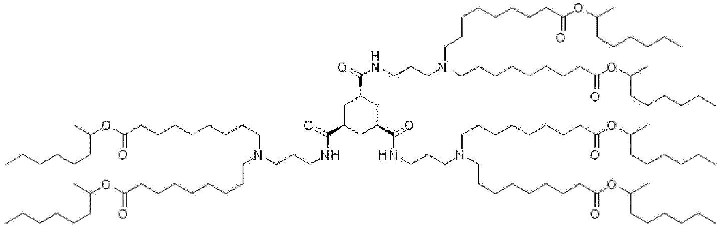 |
| 9 | 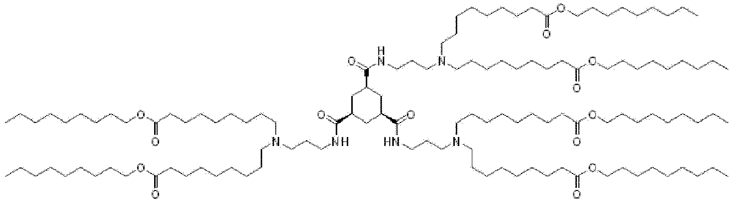 |
| 10 | 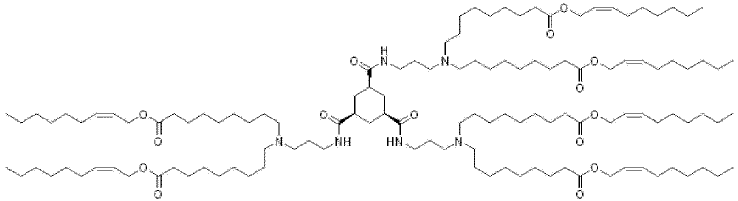 |
| 11 | 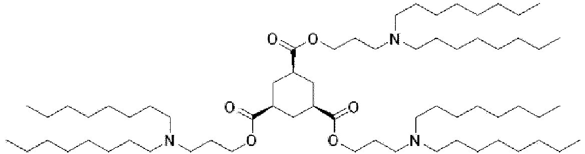 |
| 12 | 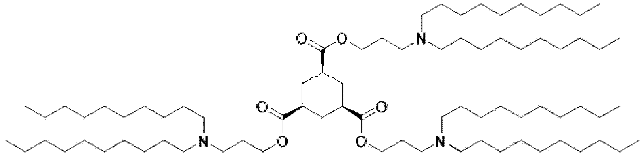 |
| 13 | 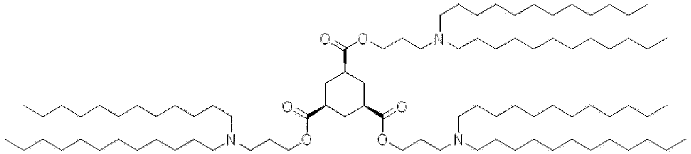 |
| 14 | 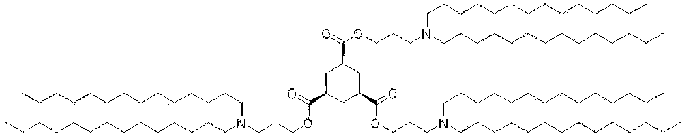 |
| 15 | 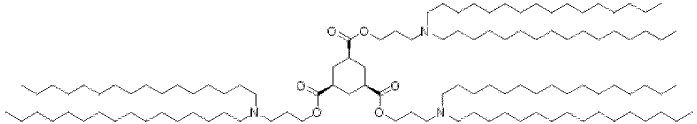 |
| 16 | 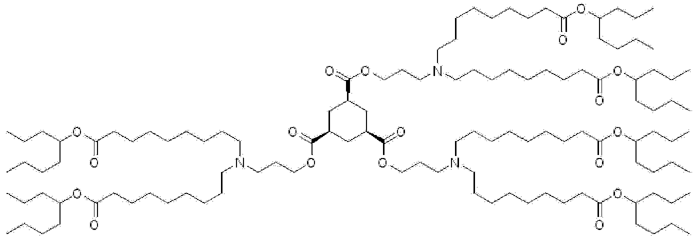 |
| 17 | 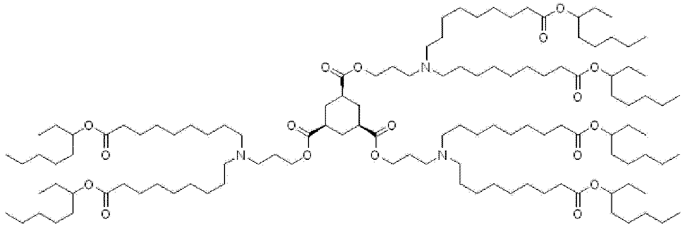 |
| 18 | 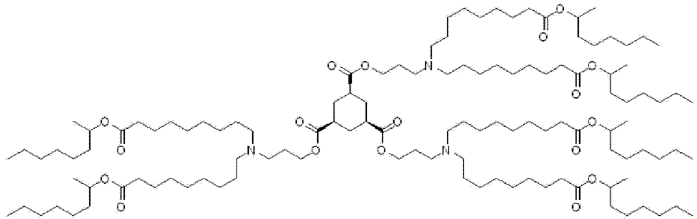 |
| 19 | 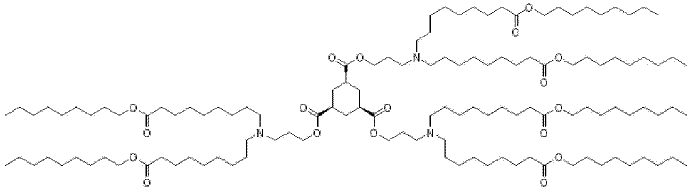 |
| 20 | 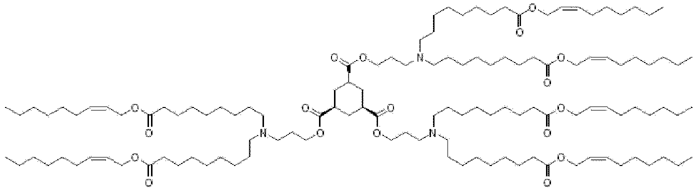 |
| 21 | 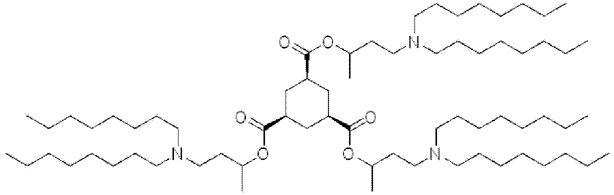 |
| 22 | 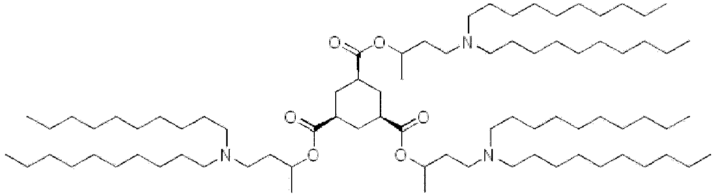 |
| 23 | 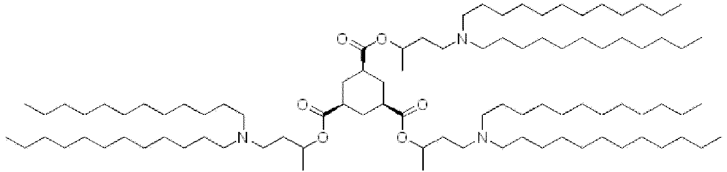 |
| 24 | 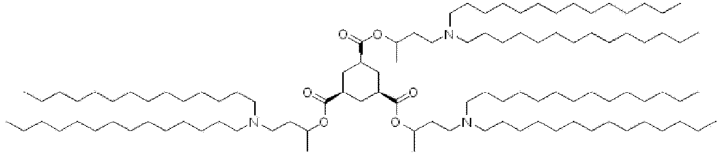 |
| 25 | 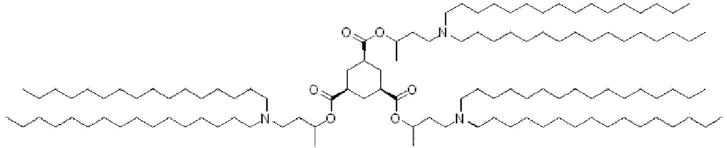 |
| 26 | 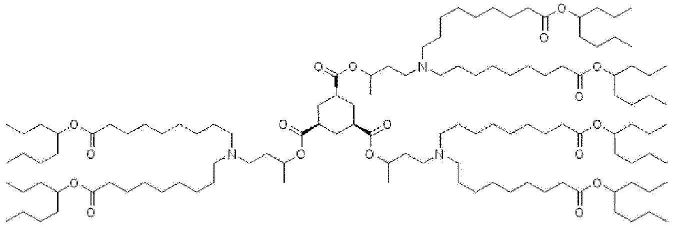 |
| 27 | 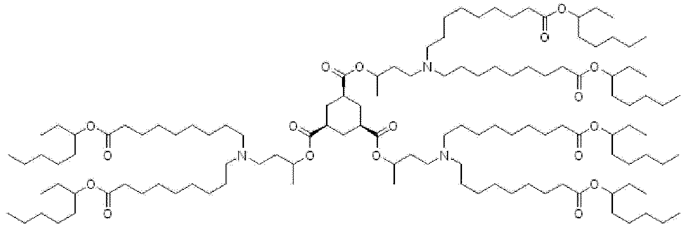 |
| 28 | 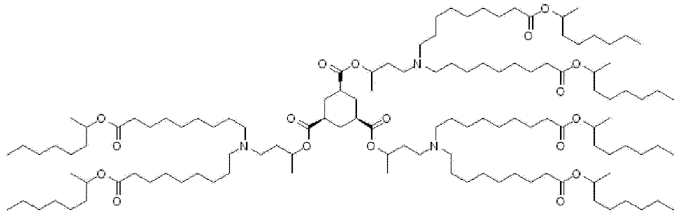 |
| 29 | 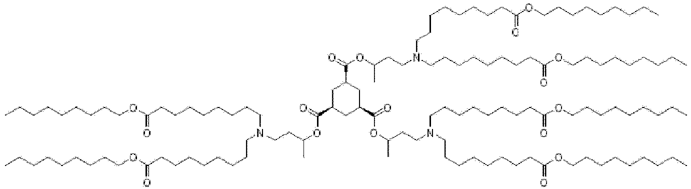 |
| 30 | 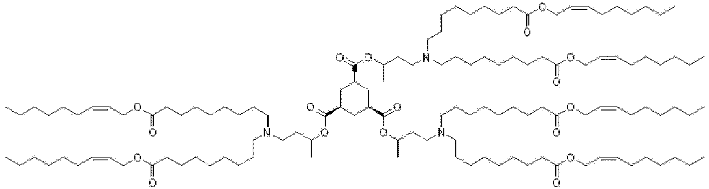 |
| 31 | 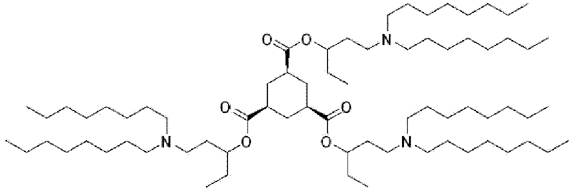 |
| 32 | 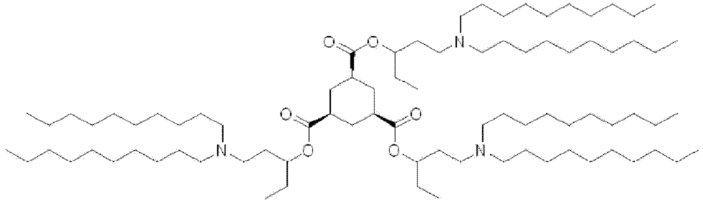 |
| 33 | 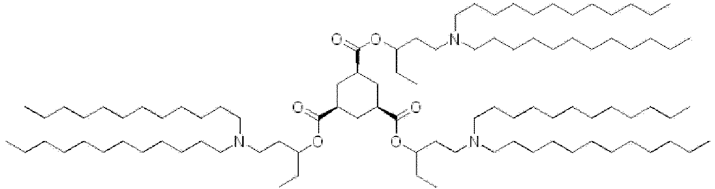 |
| 34 | 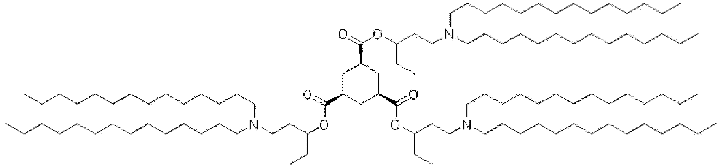 |
| 35 | 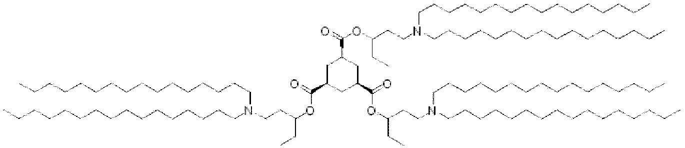 |
| 36 | 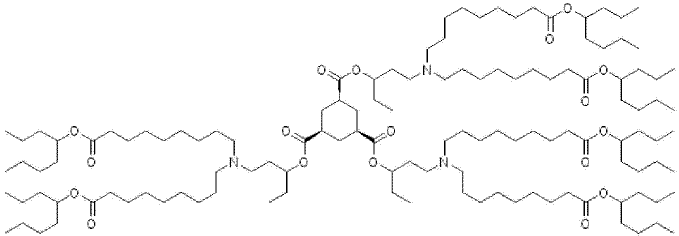 |
| 37 | 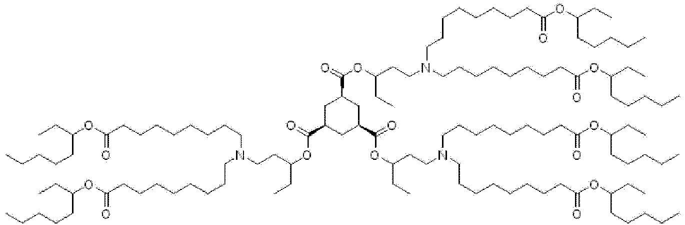 |
| 38 | 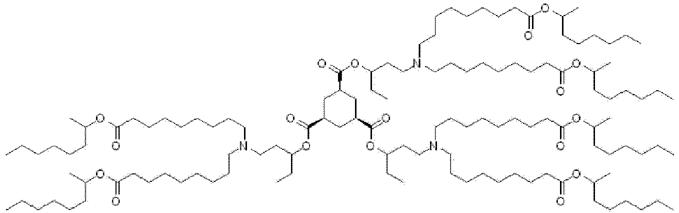 |
| 39 | 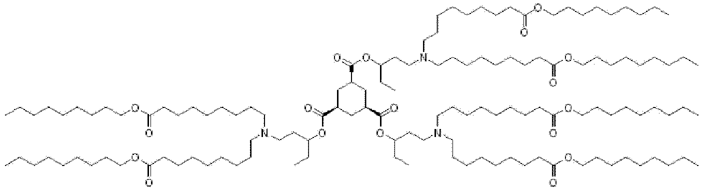 |
| 40 | 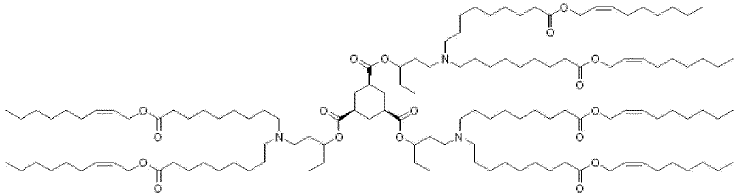 |

Hereinafter, specific examples of lipid nanoparticles comprising the above-described cycloalkane lipid compounds will be described in detail.

One embodiment of the present invention provides a lipid nanoparticle comprising a cycloalkane-based lipid compound, phospholipid, cholesterol and PEG-lipid conjugate. The cycloalkane-based lipid compound is as described above.

The phospholipid serves to surround and protect the core formed by interacting with the cycloalkane lipid compound and the drug within the lipid nanoparticle. In addition, the phospholipid binds to the phospholipid bilayer of target cells to facilitate passage of the cell membrane and endosomal escape during intracellular delivery of the drug. The phospholipid has the above-described functionality, and is not particularly limited as long as it is a substance commonly used in the relevant technical field. According to one embodiment of the present invention, the phospholipid is selected from the group consisting of distearoylphosphatidylcholine (DSPC), palmitoyloleoylphosphatidylcholine (POPC), egg phosphatidylcholine (EPC), dioleoylphosphatidylcholine (DOPC), dipalmitoylphosphatidylcholine (DPPC), dioleoylphosphatidylglycerol (DOPG), dipalmitoylphosphatidylglycerol (DPPG), distearoylphosphatidylethanolamine (DSPE), phosphatidylethanolamine (PE), dipalmitoylphosphatidylethanolamine, 1,2-dioleoyl-sn-glycero-3-phosphoethanolamine (DOPE), 1-palmitoyl-2-oleoyl-sn-glycero-3-phosphoethanolamine (POPE), 1-palmitoyl-2-oleoyl-sn-glycero-3-phosphocholine (POPC), 1,2-dioleoyl-sn-glycero-3-phosphoserine (DOPS), 1,2-dioctadecanoyl-sn-glycero-3-phosphoserine (DSPS) and combinations thereof. The phospholipid may be selected from those with excellent effectiveness depending on the type of nucleic acid to be delivered.

The cholesterol provides rigidity to the lipid filling in terms of shape within the lipid nanoparticle and is dispersed in the core and surface of the nanoparticle, thereby serving to improve the stability of the nanoparticle.

The PEG-lipid conjugate refers to a lipid in which a polyethyleneglycol (PEG) polymer, which is a hydrophilic polymer, is bound to one end of the lipid. The PEG-lipid conjugate not only contributes to the stability of nanoparticles in the serum within the lipid nanoparticle and serves to prevent aggregation between nanoparticles, but also protects nucleic acids from degrading enzymes during *in vivo* delivery of nucleic acids, thereby enhancing the stability of nucleic acids in the body and increasing the half-life of the drug encapsulated in the nanoparticle. In the PEG-lipid conjugate, the PEG and lipid may be conjugated according to methods commonly used in the relevant technical field, and in some cases, the PEG may be conjugated to the lipid through a linking group. According to one embodiment of the present invention, the lipid in the PEG-lipid conjugate is selected from the group consisting of ceramide, dimyristoyl glycerol (DMG), succinoyl-diacylglycerol (s-DAG), distearoylphosphatidylcholine (DSPC), distearoylphosphatidylethanolamine (DSPE), cholesterol, and combinations thereof.

The lipid nanoparticle is prepared by mixing a cycloalkane-based lipid compound, phospholipid, cholesterol and PEG-lipid conjugate to form a lipid mixture, and then mixing the lipid mixture with nucleic acids such as antisense RNA, siRNA and mRNA. Thus, the lipid nanoparticle may further comprise a nucleic acid. The specific preparation method is not particularly limited as long as general methods in the relevant technical field are used. In the lipid mixture, the cycloalkane-based lipid compound, phospholipid, cholesterol and PEG-lipid conjugate are mixed in an appropriate ratio considering the functionality of each component.

According to one embodiment of the present invention, the cycloalkane-based lipid compound is included in the lipid mixture in an amount of 10 mol% to 40 mol% based on the total number of moles of the cycloalkane-based lipid compound, phospholipid, cholesterol and PEG-lipid conjugate. Specifically, based on the total number of moles of the cycloalkane-based lipid compound, phospholipid, cholesterol and PEG-lipid conjugate, the cycloalkane-based lipid compound may be included in the lipid mixture in an amount of 10 mol% or more, 11 mol% or more, 12 mol% or more, 13 mol% or more, 14 mol% or more, or 15 mol% or more, and may be included in the lipid mixture in an amount of 40 mol% or less, 39 mol% or less, 38 mol% or less, 37 mol% or less, 36 mol% or less, 35 mol% or less, 34 mol% or less, 33 mol% or less, 32 mol% or less, 31 mol% or less, or 30 mol% or less.

According to one embodiment of the present invention, the phospholipid is included in the lipid mixture in an amount of 10 mol% to 50 mol% based on the total number of moles of the cycloalkane-based lipid compound, phospholipid, cholesterol and PEG-lipid conjugate. Specifically, based on the total number of moles of the cycloalkane-based lipid compound, phospholipid, cholesterol and PEG-lipid conjugate, the phospholipid may be included in the lipid mixture in an amount of 10 mol% or more, 11 mol% or more, 12 mol% or more, 13 mol% or more, 14 mol% or more, 15 mol% or more, 16 mol% or more, 17 mol% or more, 18 mol% or more, 19 mol% or more, or 20 mol% or more, and may be included in the lipid mixture in an amount of 50 mol% or less, 49 mol% or less, 48 mol% or less, 47 mol% or less, 46 mol% or less, 45 mol% or less, 44 mol% or less, 43 mol% or less, 42 mol% or less, 41 mol% or less, or 40 mol% or less.

According to one embodiment of the present invention, the cholesterol is included in the lipid mixture in an amount of 20 mol% to 60 mol% based on the total number of moles of the cycloalkane-based lipid compound, phospholipid, cholesterol and PEG-lipid conjugate. Specifically, based on the total number of moles of the cycloalkane-based lipid compound, phospholipid, cholesterol and PEG-lipid conjugate, the cholesterol may be included in the lipid mixture in an amount of 20 mol% or more, 21 mol% or more, 22 mol% or more, 23 mol% or more, 24 mol% or more, 25 mol% or more, 26 mol% or more, 27 mol% or more, 28 mol% or more, 29 mol% or more, or 30 mol% or more, and may be included in the lipid mixture in an amount of 60 mol% or less, 59 mol% or less, 58 mol% % or less, 57 mol% or less, 56 mol% or less, 55 mol% or less, 54 mol% or less, 53 mol% or less, 52 mol% or less, 51 mol% or less, or 50 mol% or less.

According to one embodiment of the present invention, the PEG-lipid conjugate is included in the lipid mixture in an amount of 0.2 mol% to 3.0 mol% based on the total number of moles of the cycloalkane-based lipid compound, phospholipid, cholesterol and PEG-lipid conjugate. Specifically, based on the total number of moles of the cycloalkane-based lipid compound, phospholipid, cholesterol and PEG-lipid conjugate, the PEG-lipid conjugate may be included in the lipid mixture in an amount of 0.2 mol% or more, 0.25 mol% or more, 0.3 mol% or more, 0.35 mol% or more, 0.4 mol% or more, 0.45 mol% or more, or 0.5 mol% or more, and may be included in the lipid mixture in an amount of 3.0 mol% or less, 2.5 mol% or less, 2 mol% or less, 1.5 mol% or less, or 1 mol% or less.

According to one embodiment of the present invention, the nucleic acid included in the lipid nanoparticle is selected from the group consisting of small interfering ribonucleic acid (siRNA), ribosomal ribonucleic acid (rRNA), ribonucleic acid (RNA), deoxyribonucleic acid (DNA), complementary deoxyribonucleic acid (cDNA), Self-amplifying RNA(SAM), Circular RNA, aptamer, messenger ribonucleic acid (mRNA), transfer ribonucleic acid (tRNA), antisense oligonucleotide, shRNA, miRNA, ribozyme, PNA, DNAzyme, and sgRNA for gene editing, and combinations thereof.

### [Best Mode]

### EXAMPLES

### Preparation of compounds:

Hereinafter, specific preparation methods for Compounds 1 to 40 in Table 1 above will be described.

Compounds 1 to 40 according to one embodiment of the present invention are basically prepared according to following Scheme 1:

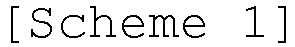

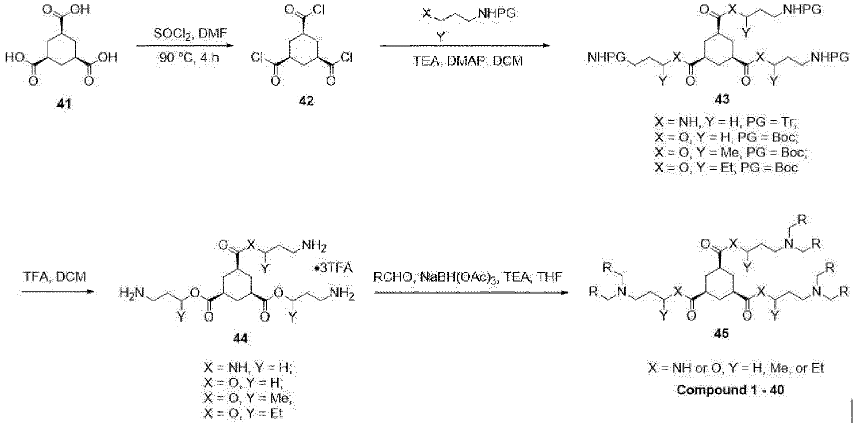

Compound 45 finally prepared in Scheme 1 above may be specified to Compounds 1 to 40 depending on the kind of R. In Scheme 1 above, the amine of Compound 44 reacts with aldehyde (RCHO) to introduce an R group into the amine. When the R group includes an ester group as in Compound 6 and the like, it may be obtained through following Scheme 2:

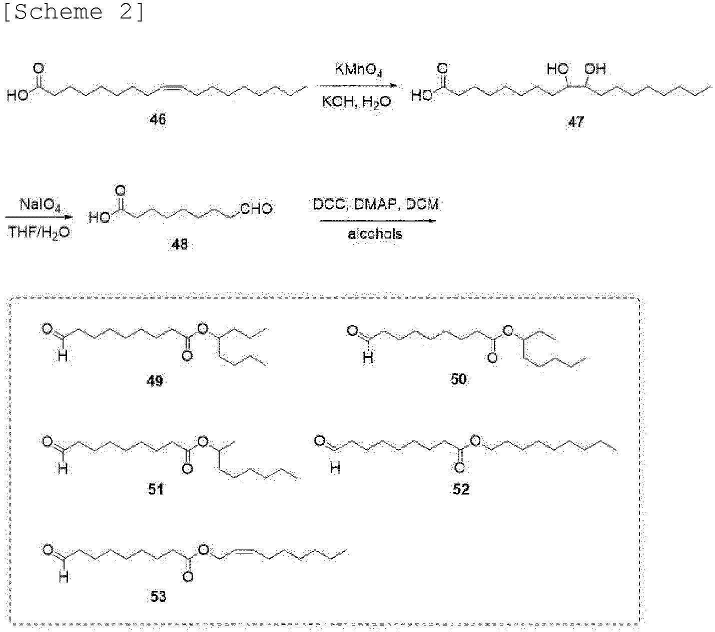

Specifically, Compounds 1 to 40 in SOCl₂ (8 mL) were added to a solution of *cis,cis*-1,3,5-cyclohexanetricarboxylic acid (Compound 41) (3.2 mmol, 691.8 mg, 1.0 equiv) with one drop of dimethylformamide (DMF) at room temperature, and then the reaction mixture was warmed to 90°C and stirred for 3 hours. Excess SOCl₂ was removed under reduced pressure, and the residue was co-evaporated with toluene to obtain *cis,cis*-1,3,5-tricarbonyl chloride compound (Compound 42) in quantitative yield. The *cis,cis*-1,3,5-tricarbonyl chloride compound (Compound 42) was dissolved in anhydrous dichloromethane (DCM) (6 mL) and added dropwise to a solution of amine (Compound 54) or alcohol (Compounds 55 to 57) (3.15 equiv, 10.0 mmol) and triethylamine (6.0 equiv, d=0.728, 19.2 mmol, 2.67 mL) in DCM (6 mL). In this case, the amine (Compound 54) or alcohol (Compounds 55 to 57) are the following compounds:

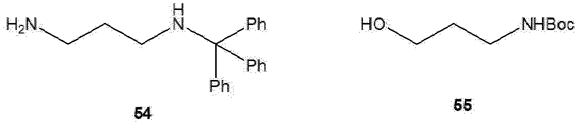

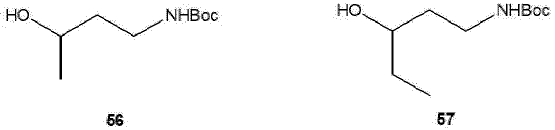

The reaction mixture was stirred at 0°C for 1 hour, and then stirred at room temperature overnight. The reaction mixture was then diluted with DCM (50 mL), washed with 1 M HCl (2×15 mL), dried over Na₂SO₄, and concentrated in vacuo to give a solid. The residue was purified by column chromatography using a CombiFlash Rf system with a RediSep Gold Resolution silica column (Teledyne Isco) with gradient elution from 100% hexane to hexane/EA (50/50 by volume) to give Compounds 58 to 61.

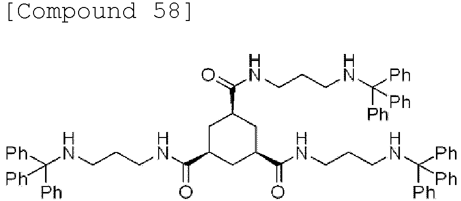

### ¹H NMR and MS results:

¹H NMR (300 MHz, DMSO-d) δ 10.50 (br. s, 3H), 8.10 (br. s, 3H), 7.41 - 7.32 (m, 45H), 3.15 - 2.97 (m, 6H), 2.82 - 2.61 (m, 6H), 2.07 - 1.93 (m, 9H), 1.42 (d, *J* = 11.2 Hz, 3H), 1.26 - 1.17 (m, 3H). MS (ESI, *m*/*z*) : [M+H]⁺ calcd. For C₇₅H₇₉N₆O₃, 1111.6; found: 1111.7.

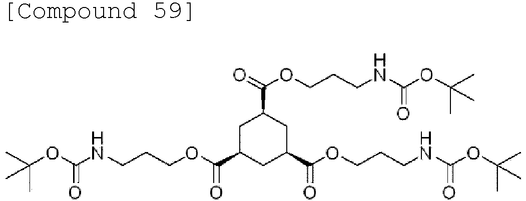

### ¹H NMR and MS results:

¹H NMR (300 MHz, Chloroform-d) δ 4.70 (s, 3H), 4.15 (t, *J* = 6.3 Hz, 6H), 3.18 (q, *J* = 6.3 Hz, 6H), 2.39 (tt, *J* = 12.5, 3.6 Hz, 3H), 2.26 (dd, *J* = 13.2, 3.6 Hz, 3H), 1.82 (p, *J* = 6.6 Hz, 6H), 1.53 (q, *J* = 12.9 Hz, 3H), 1.43 (s, 27H). MS (ESI, *m*/*z*): [M+H]⁺ calcd. For C₃₃H₅₈N₃O₁₂, 688.4; found: 688.5.

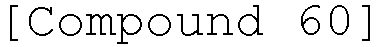

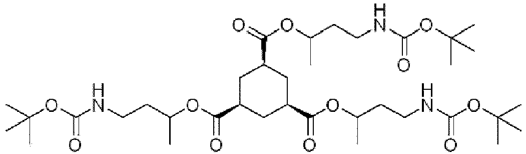

### ¹H NMR and MS results:

¹H NMR (300 MHz, Chloroform-*d*) δ 4.97 (h, *J* = 6.3 Hz, 3H), 4.79 (br s, 3H), 3.22 (m, 3H), 2.97 (m, 3H), 2.35 (tt, *J* = 12.3, 3.3 Hz, 3H), 2.22 (br d, *J* = 13.2 Hz, 3H), 1.70 (q, *J* = 6.8 Hz, 6H), 1.51 (q, *J* = 16.8 Hz, 3H), 1.41 (s, 27H), 1.22 (d, *J =* 6.3 Hz, 9H). MS (ESI, *m*/*z*) : [M+H]⁺ calcd. For C₃₆H₆₄N₃O₁₂, 730.4; found: 730.6.

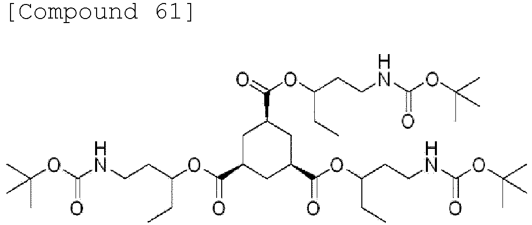

### ¹H NMR and MS results:

1H NMR (300 MHz, Chloroform-d) δ 5.08 - 4.45 (m, 6H), 3.42 - 3.16 (m, 3H), 3.04 - 2.82 (m, 3H), 2.56 - 2.31 (m, 3H), 2.33 - 2.08 (m, 3H), 1.83 - 1.45 (m, 15H), 1.45 - 1.37 (m, 27H), 0.98 - 0.83 (m, 9H). MS (ESI, *m*/*z*) : [M+H]⁺ calcd. For C₃₉H₇₀N₃O₁₂, 772.5; found: 772.5.

Trifluoroacetic acid (TFA) (0.5 mL) was added to a solution of core amine compounds (Compounds 54 to 57) (0.1 mmol, 1.0 equiv) in DCM (1.5 mL). The mixture was stirred at room temperature for 2 hours, and then the solvent and excess TFA were evaporated, and the residue was dissolved in methanol (MeOH) and concentrated three times. Aldehyde (0.8 mmol, 8.0 equiv) followed by NaBH(OAc)₃ (170 mg, 0.8 mmol, 8.0 equiv) and triethylamine (TEA) (42 µL, 0.3 mmol, 3.0 equiv) were added to a suspension of the residue obtained in the above step in 10 mL of anhydrous tetrahydrofuran, and the resulting reaction mixture was stirred at room temperature for 48 hours. In this case, as the aldehyde, alkyl aldehydes having 8, 10, 12, 14 or 16 carbon atoms, or aldehydes of Compounds 49 to 53 were used. The reaction was quenched with saturated aqueous NaHCO₃ solution and extracted with DCM (3×25 mL), and then the organic phases were combined, washed with 60 mL of 1 M NaHCO₃, and dried over anhydrous Na₂SO₄. The solution was filtered and the solvent was removed under reduced pressure. The residue was purified by column chromatography using a CombiFlash Rf system with a RediSep Gold Resolution silica column (Teledyne Isco) with gradient elution from 100% CH₂Cl₂ to CH₂Cl₂/MeOH/NH₄OH (75/22/3 by volume) to give Compounds 1 to 40.

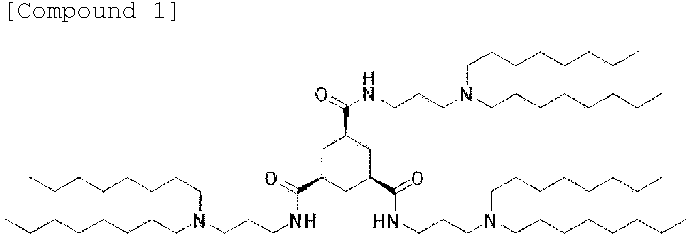

### ¹H NMR results:

¹H NMR (300 MHz, Chloroform-d) δ 7.26 (s, 3H), 3.43 - 3.08 (m, 6H), 2.44 (dt, *J =* 27.4, 7.2 Hz, 21H), 2.04 (d, *J* = 11.8 Hz, 3H), 1.62 (p, *J* = 6.6 Hz, 9H), 1.41 (t, *J* = 7.5 Hz, 12H), 1.35 - 1.15 (m, 60H), 0.87 (t, *J* = 6.6 Hz, 18H).

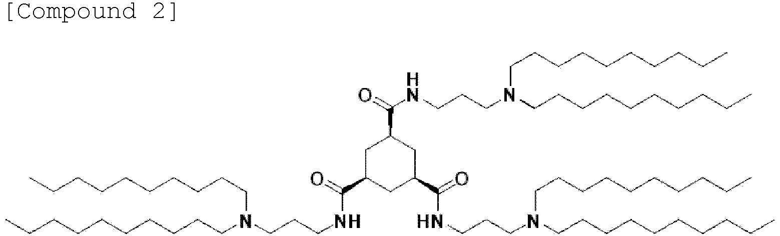

### ¹H NMR results:

¹H NMR (300 MHz, Chloroform-d) δ 7.06 (s, 3H), 3.28 (q, *J* = 6.0 Hz, 6H), 2.48 (dt, *J* = 26.8, 7.2 Hz, 18H), 2.25 - 2.13 (m, 3H), 2.07 (d, *J* = 13.1 Hz, 3H), 1.73 - 1.52 (m, 9H), 1.45 (q, *J* = 7.7 Hz, 12H), 1.35 - 1.17 (m, 84H), 0.87 (t, *J* = 6.6 Hz, 18H).

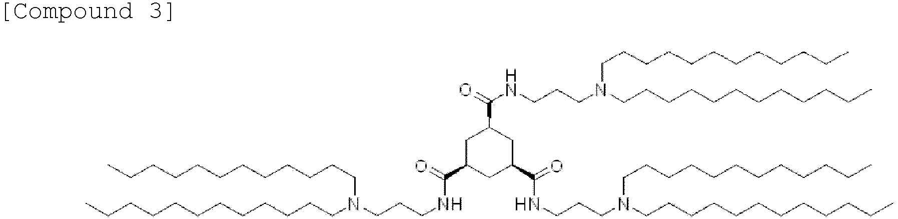

### ¹H NMR results:

¹H NMR (300 MHz, Chloroform-d) δ 7.09 - 6.84 (m, 2H), 3.28 (q, *J* = 6.0 Hz, 6H), 2.65 - 2.27 (m, 18H), 2.13 (dd, *J =* 33.0, 14.4 Hz, 6H), 1.60 (qd, *J* = 11.8, 6.8 Hz, 9H), 1.42 (q, *J* = 7.4 Hz, 12H), 1.34 - 1.17 (m, 108H), 0.87 (t, J = 6.6 Hz, 18H).

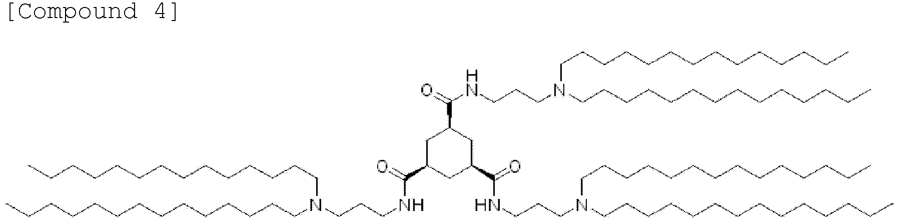

### ¹H NMR results:

¹H NMR (300 MHz, Chloroform-d) δ 7.02 (t, *J* = 4.9 Hz, 3H), 3.28 (q, *J* = 6.0 Hz, 6H), 2.50 (t, *J* = 6.3 Hz, 6H), 2.47 - 2.34 (m, 12H), 2.25 - 1.99 (m, 6H), 1.73 - 1.49 (m, 9H), 1.43 (q, *J* = 7.6 Hz, 12H), 1.35 - 1.15 (m, 132H), 0.87 (t, *J* = 6.6 Hz, 18H).

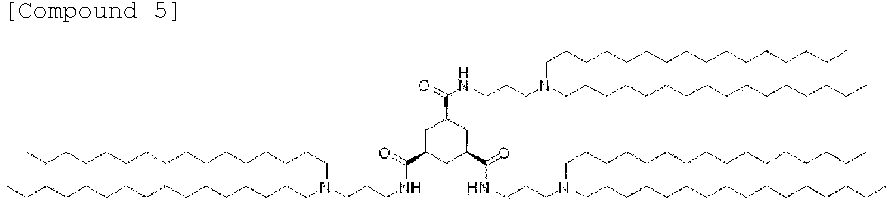

### ¹H NMR results:

¹H NMR (300 MHz, Chloroform-d) δ 6.95 (t, *J* = 5.0 Hz, 3H), 3.29 (q, *J* = 6.0 Hz, 6H), 2.46 (ddd, *J* = 25.9, 10.3, 5.6 Hz, 18H) , 2.11 (t, *J* = 11.7 Hz, 6H), 1.72 - 1.52 (m, 9H), 1.43 (t, *J* = 7.7 Hz, 12H), 1.35 - 1.14 (m, 156H), 0.88 (t, *J* = 6.6 Hz, 18H) .

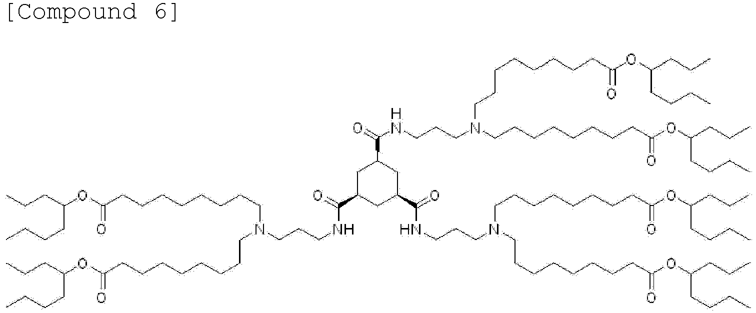

### ¹H NMR results:

¹H NMR (300 MHz, Chloroform-d) δ 6.78 (t, *J* = 5.1 Hz, 3H), 4.87 (p, *J* = 6.3 Hz, 6H), 3.27 (q, *J* = 6.1 Hz, 6H), 2.44 (dt, *J* = 25.0, 7.2 Hz, 18H), 2.27 (t, *J* = 7.5 Hz, 12H), 2.20 - 1.99 (m, 6H), 1.68 - 1.36 (m, 57H), 1.36 - 1.16 (m, 84H), 0.88 (td, *J* = 7.2, 4.8 Hz, 36H).

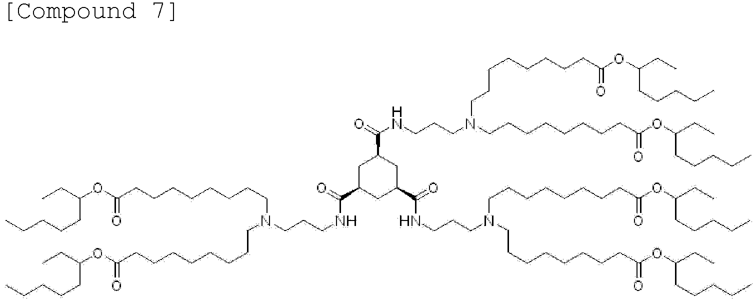

### ¹H NMR results:

¹H NMR (300 MHz, Chloroform-d) δ 6.85 (t, *J* = 5.2 Hz, 2H), 4.80 (p, *J* = 6.2 Hz, 6H), 3.27 (q, *J* = 6.1 Hz, 6H), 2.44 (dt, J = 25.3, 7.2 Hz, 18H), 2.27 (t, *J* = 7.5 Hz, 12H), 2.21 - 2.01 (m, 6H), 1.70 - 1.36 (m, 57H), 1.34 - 1.16 (m, 84H), 0.95 - 0.79 (m, 36H).

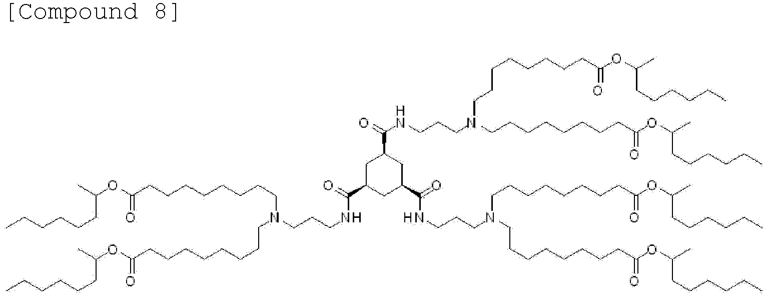

### ¹H NMR results:

¹H NMR (300 MHz, Chloroform-d) δ 6.81 (s, 2H), 5.00 - 4.79 (m, 6H), 3.28 (q, *J* = 6.1 Hz, 6H), 2.60 - 2.34 (m, 18H), 2.26 (t, *J* = 7.5 Hz, 12H), 2.21 - 2.01 (m, 6H), 1.75 - 1.38 (m, 45H), 1.37 - 1.15 (m, 114H), 0.93 - 0.81 (m, 18H).

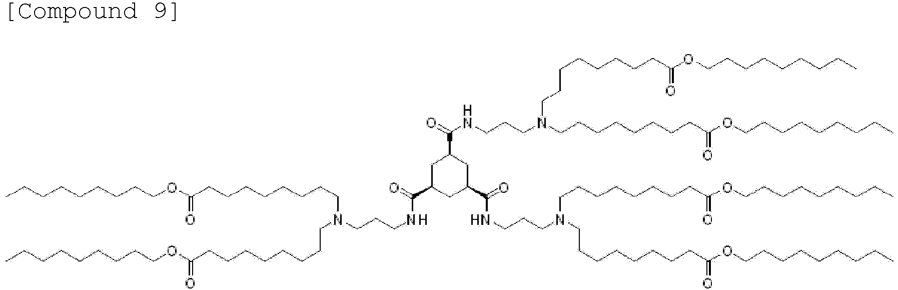

### ¹H NMR results:

¹H NMR (300 MHz, Chloroform-*d*) δ 6.92 (s, 2H), 4.04 (t, *J* = 6.7 Hz, 12H), 3.28 (q, *J* = 6.1 Hz, 6H), 2.50 (d, *J =* 22.8 Hz, 18H), 2.28 (t, *J* = 7.5 Hz, 12H), 2.14 (dd, *J* = 31.0, 13.6 Hz, 6H), 1.61 (p, *J* = 7.1 Hz, 33H), 1.45 (s, 12H), 1.37 - 1.15 (m, 120H), 0.87 (t, *J* = 6.6 Hz, 18H).

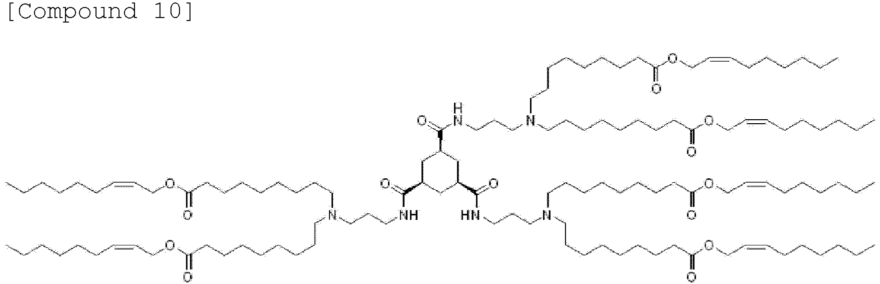

### ¹H NMR results:

¹H NMR (300 MHz, Chloroform-*d*) δ 6.89 (d, *J* = 6.3 Hz, 3H), 5.63 (dt, *J* = 11.1, 7.3 Hz, 6H), 5.57 - 5.44 (m, 6H), 4.61 (d, *J* = 6.7 Hz, 12H), 3.28 (q, *J* = 6.1 Hz, 6H), 2.48 (dd, *J* = 23.9, 7.3 Hz, 18H) , 2.29 (t, *J* = 7.5 Hz, 12H), 2.09 (q, *J* = 6.8 Hz, 18H), 1.60 (h, *J* = 6.8, 6.4 Hz, 21H), 1.50 - 1.18 (m, 108H), 0.87 ((t, *J* = 6.6 Hz, 18H).

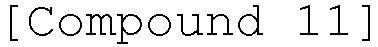

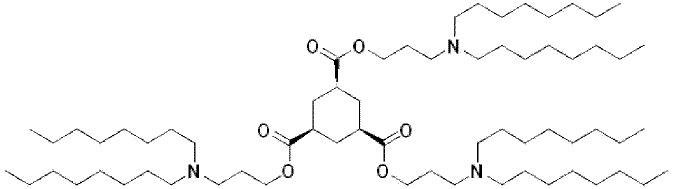

### ¹H NMR results:

¹H NMR (300 MHz, Chloroform-d) δ 4.12 (t, *J* = 6.6 Hz, 6H), 2.75 - 2.28 (m, 21H), 2.25 (dt, *J* = 12.9, 3.5 Hz, 3H), 1.77 (t, *J* = 7.4 Hz, 6H), 1.55 (t, *J* = 12.8 Hz, 3H), 1.47 - 1.34 (m, 12H), 1.32 - 1.20 (m, 60H), 0.88 (t, *J* = 6.6 Hz, 18H).

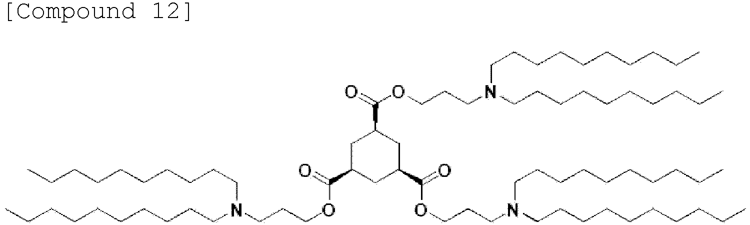

### ¹H NMR results:

¹H NMR (300 MHz, Chloroform-d) δ 4.12 (t, *J* = 6.5 Hz, 6H), 2.69 - 2.28 (m, 21H), 2.28 - 2.17 (m, 3H), 1.79 (s, 6H), 1.60 - 1.35 (m, 15H), 1.34 - 1.18 (s, 84H), 0.88 (t, J = 6.6 Hz, 18H).

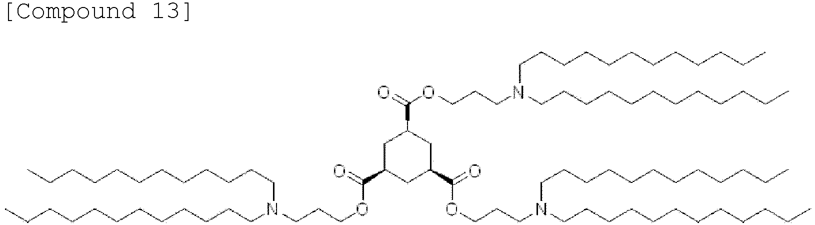

### ¹H NMR results:

¹H NMR (300 MHz, Chloroform*-d*) δ 4.12 (t, *J* = 6.5 Hz, 6H), 3.04 - 2.27 (m, 21H), 2.25 (d, *J* = 13.3 Hz, 3H), 1.73 (d, *J* = 36.2 Hz, 6H), 1.60 - 1.32 (m, 16H), 1.32 - 1.20 (m, 108H), 0.88 (t, J = 6.6 Hz, 18H).

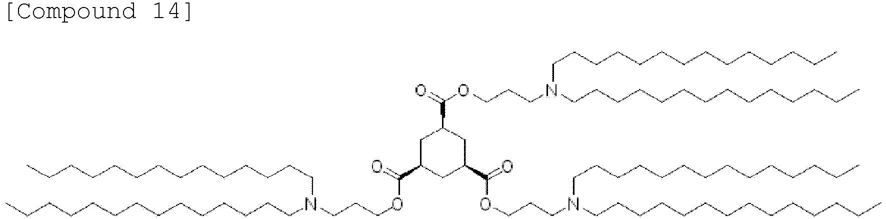

### ¹H NMR results:

¹H NMR (300 MHz, Chloroform-d) δ 4.13 (t, *J* = 6.5 Hz, 6H), 2.92 - 2.28 (m, 21H), 2.25 (d, *J* = 13.3 Hz, 3H), 1.84 (s, 6H), 1.52 (q, *J* = 12.6 Hz, 15H), 1.34 - 1.16 (m, 134H), 0.88 (t, *J* = 6.6 Hz, 18H).

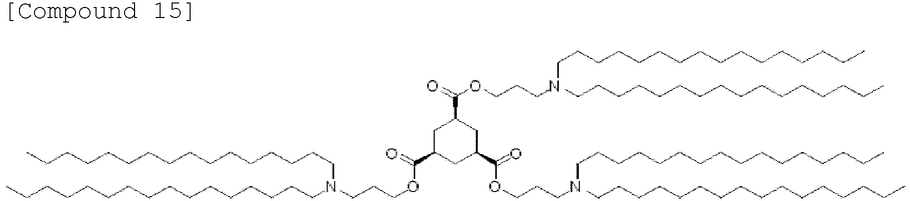

### ¹H NMR results:

¹H NMR (300 MHz, Chloroform-d) δ 4.12 (t, *J* = 6.6 Hz, 6H), 2.98 - 2.27 (m, 21H), 2.23 (d, *J* = 3.3 Hz, 3H), 1.80 (s, 6H), 1.52 (dd, *J* = 27.0, 14.1 Hz, 15H), 1.32 - 1.19 (m, 156H), 0.88 (t, *J* = 6.6 Hz, 18H).

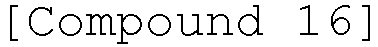

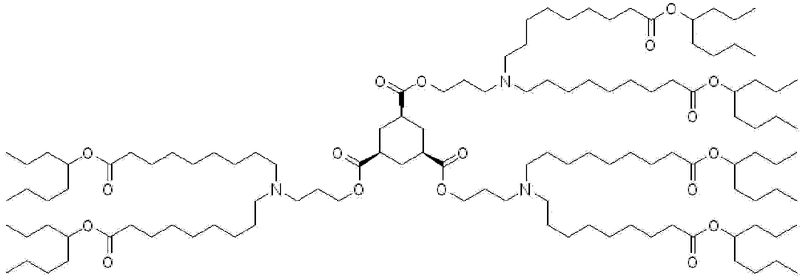

### ¹H NMR results:

¹H NMR (300 MHz, Chloroform-d) δ 4.88 (p, *J* = 6.2 Hz, 6H), 4.17 (d, *J* = 15.8 Hz, 6H), 3.16 - 2.31 (m, 21H), 2.27 (t, *J* = 7.5 Hz, 15H), 1.90 - 1.71 (m, 6H), 1.71 - 1.36 (m, 51H), 1.37 - 1.10 (m, 84H), 0.89 (td, *J* = 7.1, 4.8 Hz, 36H).

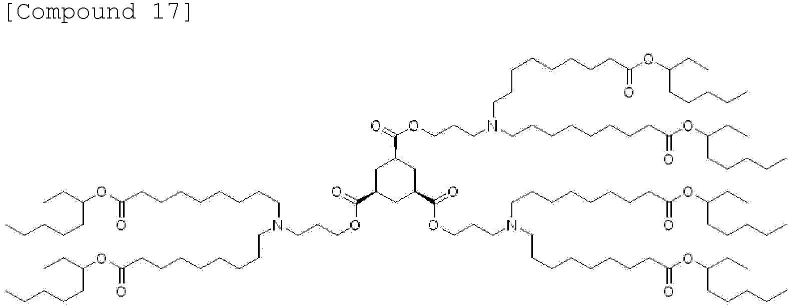

### ¹H NMR results:

¹H NMR (300 MHz, Chloroform-d) δ 4.80 (p, *J* = 6.3 Hz, 6H), 4.10 (t, *J* = 6.6 Hz, 6H), 2.94 - 2.30 (m, 21H), 2.26 (d, *J* = 7.5 Hz, 12H), 2.22 (d, *J* = 3.4 Hz, 3H), 1.76 (s, 6H), 1.66 - 1.35 (m, 51H), 1.35 - 1.17 (m, 84H), 0.94 - 0.79 (m, 36H).

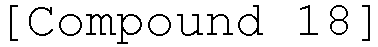

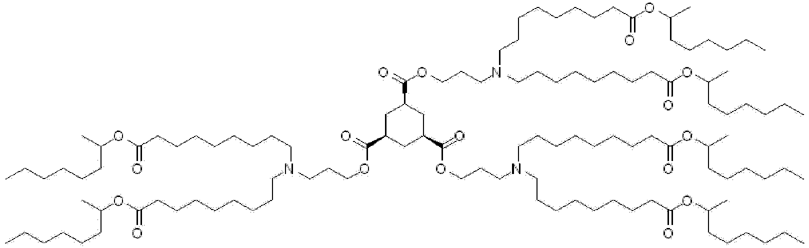

### ¹H NMR results:

¹H NMR (300 MHz, Chloroform-*d*) δ 5.01 - 4.79 (m, 6H), 4.11 (t, *J* = 6.6 Hz, 6H), 2.93 - 2.28 (m, 21H), 2.25 (t, *J* = 7.5 Hz, 15H), 1.78 (s, 6H), 1.66 - 1.35 (m, 39H), 1.34 - 1.21 (m, 95H), 1.18 (d, *J* = 6.3 Hz, 18H), 0.92 - 0.82 (m, 18H).

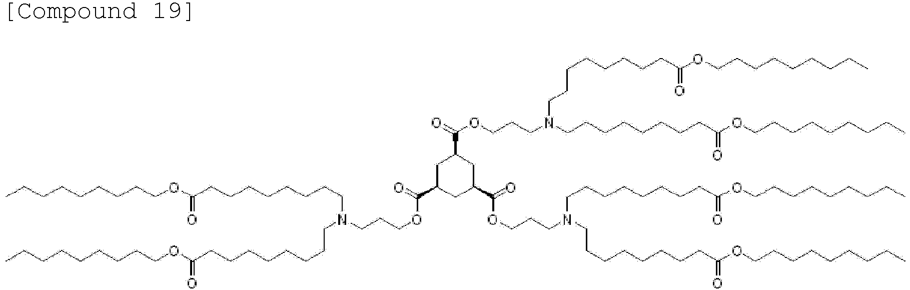

### ¹H NMR results:

¹H NMR (300 MHz, Chloroform-*d*) δ 4.11 (t, *J* = 6.6 Hz, 6H), 4.04 (t, *J* = 6.7 Hz, 12H), 2.79 - 2.08 (m, 36H), 1.78 (s, 6H), 1.59 (h, *J* = 6.3, 5.8 Hz, 27H), 1.44 (d, *J* = 9.4 Hz, 6H), 1.37 - 1.20 (m, 125H), 0.87 (t, *J* = 6.3 Hz, 18H).

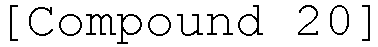

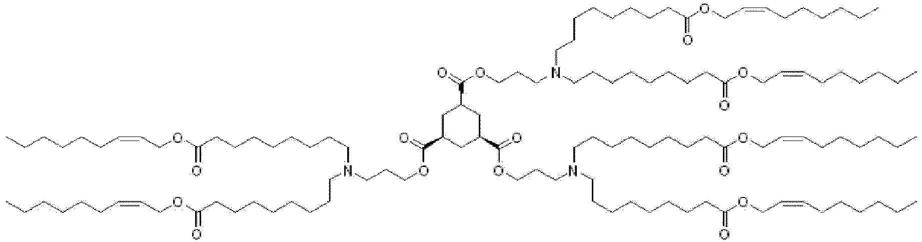

### ¹H NMR results:

¹H NMR (300 MHz, Chloroform-d) δ 5.63 (dt, *J* = 11.1, 7.4 Hz, 6H), 5.56 - 5.45 (m, 6H), 4.60 (d, *J* = 6.7 Hz, 12H), 4.10 (t, *J* = 6.6 Hz, 6H), 2.66 - 2.32 (m, 19H), 2.29 (t, *J* = 7.6 Hz, 14H), 2.22 (dd, *J* = 9.0, 5.5 Hz, 3H), 2.08 (q, *J* = 7.1 Hz, 12H), 1.76 (s, 6H), 1.66 - 1.38 (m, 27H), 1.36 - 1.19 (m, 96H), 0.87 (t, *J* = 6.6 Hz, 18H).

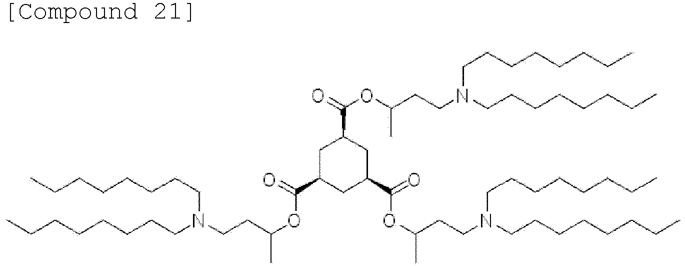

### ¹H NMR results:

¹H NMR (300 MHz, Chloroform-d) δ 5.08 - 4.86 (m, 3H), 2.80 - 2.27 (m, 21H), 2.24 (d, *J* = 13.3 Hz, 3H), 1.75 (dp, *J* = 21.1, 7.8, 7.2 Hz, 6H), 1.61 - 1.36 (m, 15H), 1.36 - 1.18 (d, *J* = 12.2 Hz, 69H), 0.88 (t, *J* = 6.6 Hz, 18H).

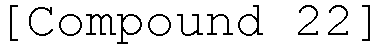

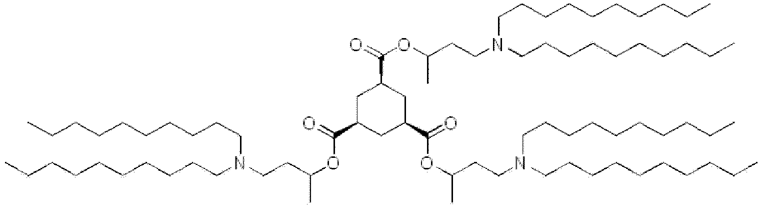

### ¹H NMR results:

¹H NMR (300 MHz, Chloroform-*d*) δ 4.93 (q, *J* = 6.3 Hz, 3H), 2.84 - 2.26 (m, 21H), 2.21 (d, *J* = 13.2 Hz, 3H), 1.79 - 1.55 (m, 6H), 1.62 - 1.31 (m, 15H), 1.32 - 1.15 (m, 93H), 0.88 (t, *J* = 6.6 Hz, 18H).

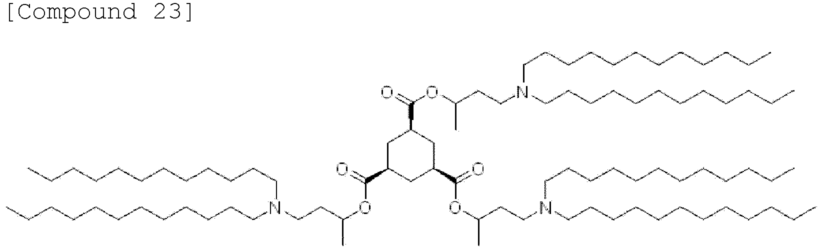

### ¹H NMR results:

¹H NMR (300 MHz, Chloroform-*d*) δ 5.04 - 4.84 (m, 3H), 2.74 - 2.26 (m, 21H), 2.21 (d, *J* = 13.4 Hz, 3H), 1.79 - 1.57 (m, 6H), 1.54 - 1.33 (m, 15H), 1.33 - 1.18 (m, 117H), 0.88 (t, *J* = 6.6 Hz, 18H).

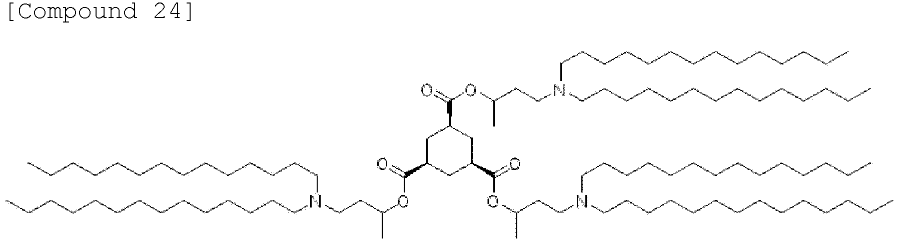

### ¹H NMR results:

¹H NMR (300 MHz, Chloroform*-d*) δ 5.16 - 4.80 (m, 3H), 2.80 - 2.24 (m, 21H), 2.21 (d, *J* = 13.1 Hz, 3H), 1.73 (d, *J* = 26.8 Hz, 6H), 1.46 (d, *J* = 15.7 Hz, 15H), 1.35 - 1.15 (m, 141H), 0.87 (t, *J* = 6.6 Hz, 18H).

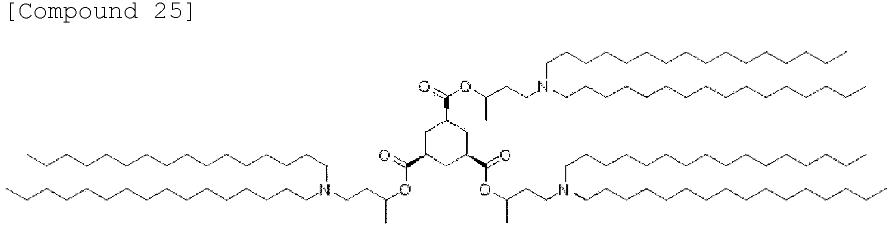

### ¹H NMR results:

¹H NMR (300 MHz, Chloroform*-d*) δ 4.93 (q, *J* = 6.3 Hz, 3H), 2.95 - 2.26 (m, 21H), 2.21 (d, *J* = 13.3 Hz, 3H), 1.86 - 1.67 (m, 6H), 1.58 - 1.35 (m, 15H), 1.35 - 1.17 (m, 165H), 0.87 (t, *J* = 6.6 Hz, 18H).

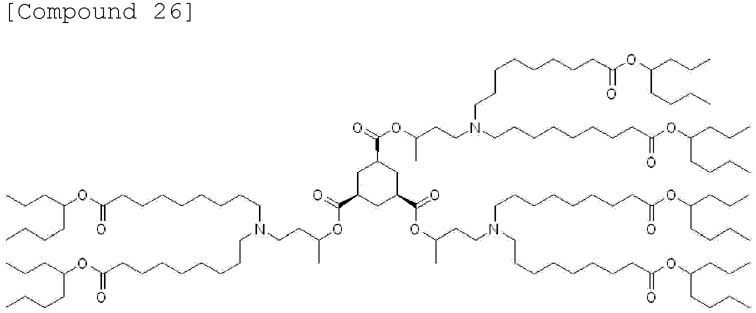

### ¹H NMR results:

¹H NMR (300 MHz, Chloroform-d) δ 4.91 (td, *J* = 12.5, 11.6, 5.5 Hz, 9H), 2.87 - 2.35 (m, 18H), 2.29 (t, *J* = 7.5 Hz, 18H), 1.75 - 1.58 (m, 18H), 1.58 - 1.39 (m, 36H), 1.38 - 1.19 (m, 96H), 0.91 (td, *J* = 7.2, 4.8 Hz, 36H).

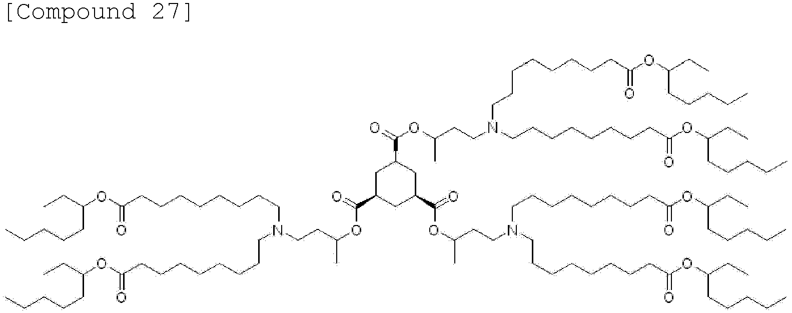

### ¹H NMR results:

¹H NMR (300 MHz, Chloroform-*d*) δ 4.92 (q, *J* = 6.3 Hz, 3H), 4.80 (p, *J* = 6.2 Hz, 6H), 2.35 (d, *J* = 14.1 Hz, 18H), 2.27 (t, *J* = 7.5 Hz, 15H), 2.20 (d, *J* = 12.5 Hz, 3H), 1.84 - 1.36 (m, 56H), 1.34 - 1.19 (m, 92H), 0.94 - 0.82 (m, 36H).

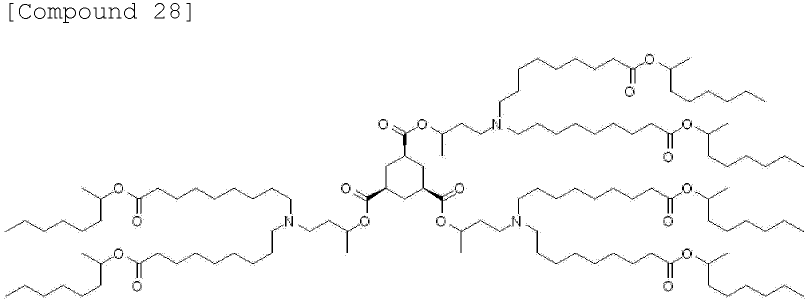

### ¹H NMR results:

¹H NMR (300 MHz, Chloroform-*d*) δ 4.89 (hept, *J* = 6.0 Hz, 9H), 2.82 - 2.31 (m, 18H), 2.25 (t, *J* = 7.5 Hz, 18H), 1.71 - 1.36 (m, 42H), 1.34 - 1.13 (m, 127H), 0.90 - 0.83 (m, 18H).

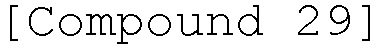

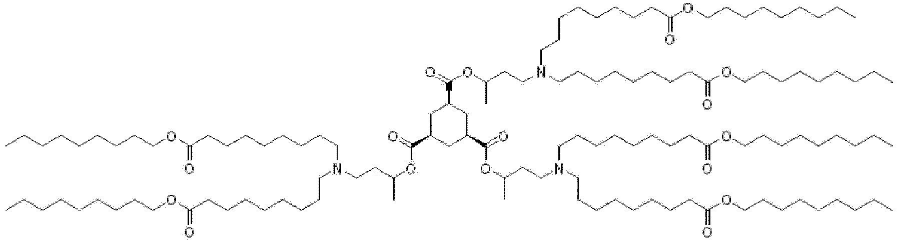

### ¹H NMR results:

¹H NMR (300 MHz, Chloroform-*d*) δ 4.94 (q, *J* = 6.3 Hz, 3H), 4.06 (t, *J* = 6.7 Hz, 12H), 2.76 - 2.34 (m, 18H), 2.30 (t, *J* = 7.5 Hz, 14H), 2.22 (d, *J* = 12.1 Hz, 3H), 1.75 - 1.55 (m, 30H), 1.55 - 1.15 (m, 144H), 0.89 (t, *J* = 6.6 Hz, 18H).

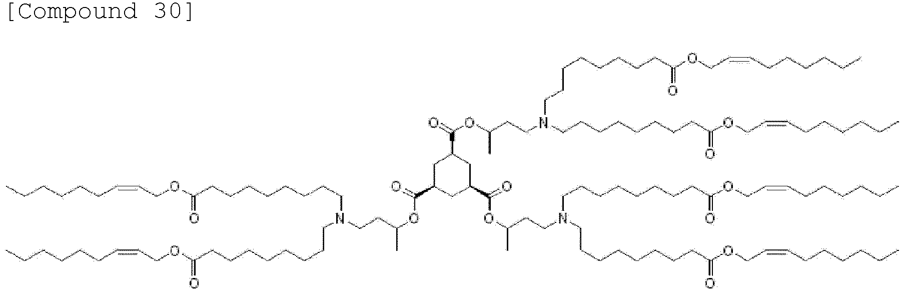

### ¹H NMR results:

¹H NMR (300 MHz, Chloroform-*d*) δ 5.64 (dt, *J* = 11.0, 7.4 Hz, 6H), 5.57 - 5.43 (m, 6H), 4.92 (q, *J* = 6.3 Hz, 3H), 4.61 (d, *J* = 6.7 Hz, 12H), 2.78 - 2.33 (m, 18H), 2.29 (t, *J* = 7.5 Hz, 15H), 2.20 (d, *J* = 12.4 Hz, 3H), 2.09 (q, *J* = 7.1 Hz, 12H), 1.70 - 1.52 (m, 18H), 1.52 - 1.13 (m, 123H), 0.87 (t, *J* = 6.6 Hz, 18H).

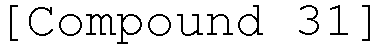

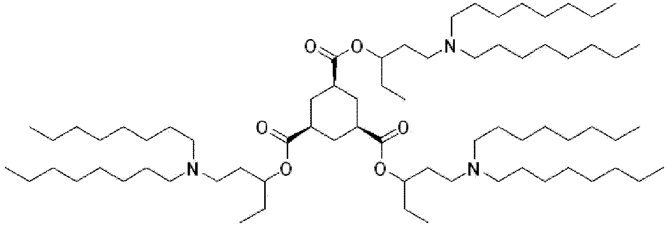

### ¹H NMR results:

¹H NMR (300 MHz, Chloroform-*d*) δ 4.97 - 4.77 (m, 3H), 2.48 (d, *J* = 51.3 Hz, 21H), 2.24 (d, *J* = 12.9 Hz, 3H), 1.84 (s, 6H), 1.65 - 1.43 (m, 18H), 1.43 - 1.12 (m, 63H), 0.97 - 0.82 (m, 27H).

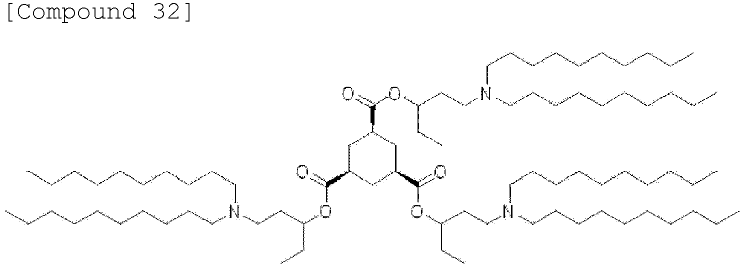

### ¹H NMR results:

¹H NMR (300 MHz, Chloroform-*d*) δ 4.85 (p, *J* = 6.3, 5.9 Hz, 3H), 2.42 (s, 21H), 2.28 - 2.17 (m, 3H), 1.80 - 1.65 (m, 6H), 1.64 - 1.54 (m, 6H), 1.53 - 1.37 (m, 12H), 1.37 - 1.15 (m, 87H), 0.93 - 0.82 (m, 27H).

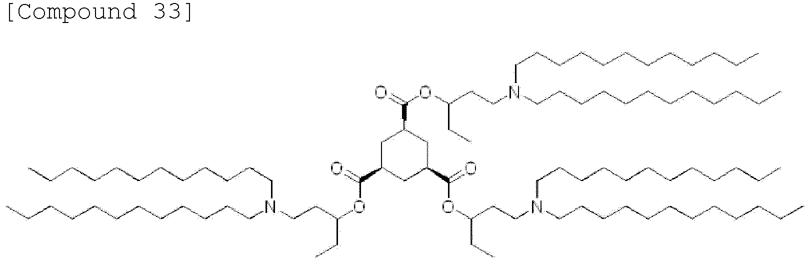

### ¹H NMR results:

¹H NMR (300 MHz, Chloroform-*d*) δ 4.84 (q, *J* = 6.0 Hz, 3H), 2.98 - 2.27 (m, 20H), 2.28 - 2.10 (m, 3H), 1.83 - 1.62 (m, 6H), 1.62 - 1.36 (m, 18H), 1.35 - 1.13 (m, 111H), 0.87 (t, *J* = 6.6 Hz, 27H).

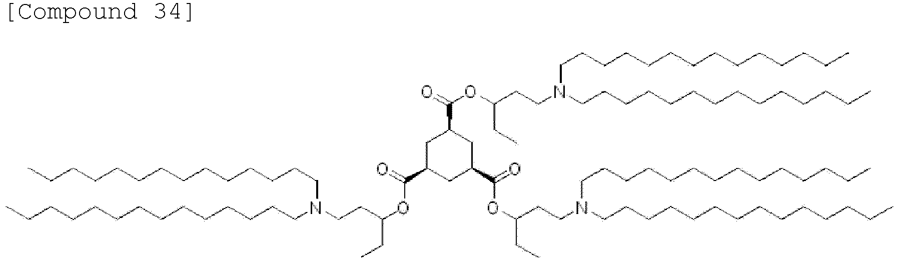

### ¹H NMR results:

¹H NMR (300 MHz, Chloroform-*d*) δ 4.85 (p, *J* = 6.2, 5.8 Hz, 3H), 2.44 (s, 21H), 2.23 (d, *J* = 12.0 Hz, 3H), 1.85 - 1.62 (m, 6H), 1.62 - 1.52 (m, 6H), 1.35 - 1.15 (m, 12H), 1.25 (s, 135H), 0.93 - 0.80 (m, 27H).

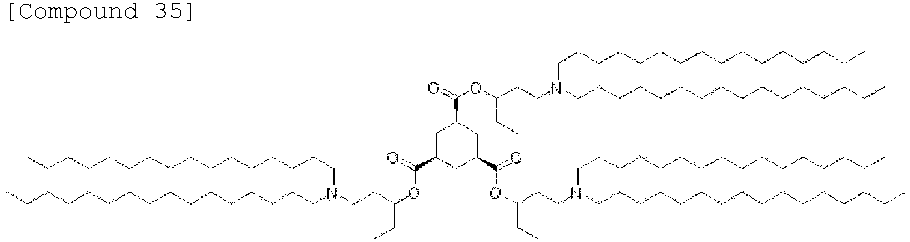

### ¹H NMR results:

¹H NMR (300 MHz, Chloroform-d) δ 5.01 - 4.69 (m, 3H), 2.91 - 2.29 (m, 21H), 2.25 (d, *J* = 12.2 Hz, 3H), 1.95 - 1.69 (m, 6H), 1.68 - 1.45 (m, 18H), 1.45 - 1.05 (m, 159H), 0.97 - 0.81 (m, 27H) .

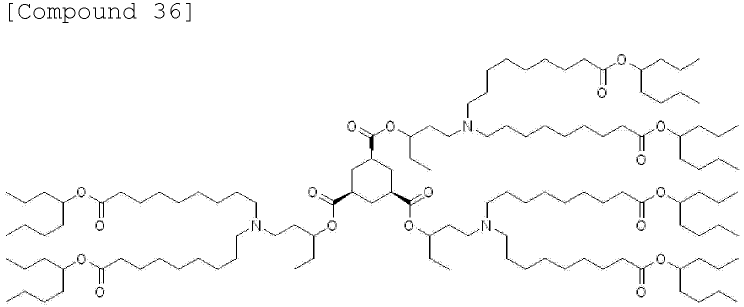

### ¹H NMR results:

¹H NMR (300 MHz, Chloroform-d) δ 4.87 (h, *J* = 5.4, 4.7 Hz, 9H), 2.98 - 2.32 (m, 21H), 2.27 (t, *J* = 7.5 Hz, 15H), 1.73 - 1.43 (m, 57H), 1.41 - 1.16 (m, 91H), 0.98 - 0.81 (m, 45H).

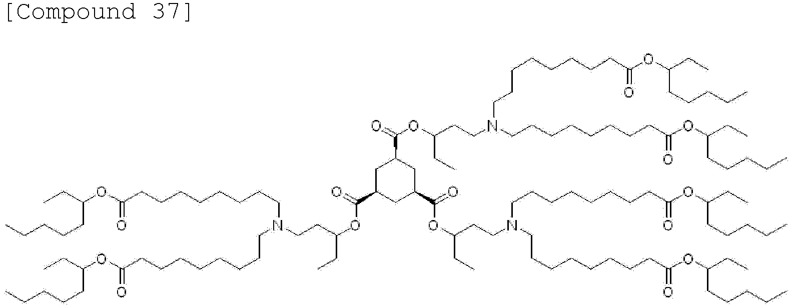

### ¹H NMR results:

¹H NMR (300 MHz, Chloroform-d) δ 4.82 (h, *J* = 6.3, 5.8 Hz, 9H), 3.05 - 2.34 (m, 21H), 2.28 (t, *J* = 7.5 Hz, 15H), 1.75 - 1.43 (m, 57H), 1.41 - 1.11 (m, 90H), 1.00 - 0.78 (m, 45H).

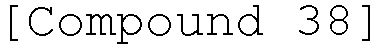

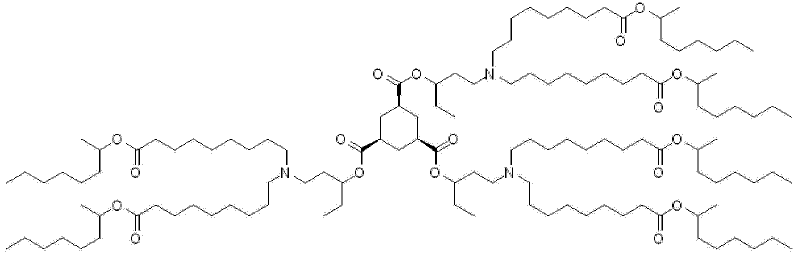

### ¹H NMR results:

¹H NMR (300 MHz, Chloroform-d) δ 5.06 - 4.66 (m, 9H), 2.87 - 2.29 (m, 21H), 2.25 (t, *J* = 7.5 Hz, 15H), 1.71 - 1.39 (m, 45H), 1.39 - 1.15 (m, 120H), 0.92 - 0.82 (m, 27H).

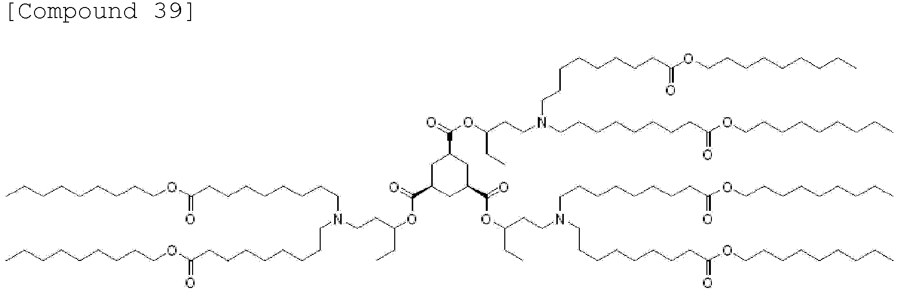

### ¹H NMR results:

¹H NMR (300 MHz, Chloroform-d) δ 4.84 (h, *J* = 5.8, 5.1 Hz, 3H), 4.05 (t, *J* = 6.7 Hz, 12H), 2.80 - 2.33 (m, 19H), 2.32 - 2.15 (m, 17H), 1.73 - 1.49 (m, 39H), 1.48 - 1.15 (m, 132H), 0.96 - 0.81 (m, 27H).

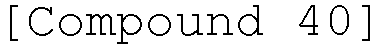

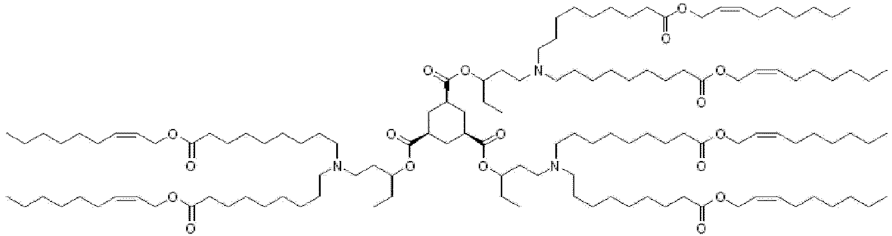

### ¹H NMR results:

¹H NMR (300 MHz, Chloroform-*d*) δ 5.64 (dt, *J* = 11.0, 7.3 Hz, 6H), 5.51 (dt, *J* = 10.9, 6.8 Hz, 6H), 4.85 (p, *J* = 6.0 Hz, 3H), 4.61 (d, *J* = 6.7 Hz, 12H), 2.81 - 2.33 (m, 19H), 2.33 - 2.16 (m, 17H), 2.09 (q, *J* = 7.1 Hz, 12H), 1.70 - 1.48 (m, 27H), 1.49 - 1.08 (m, 109H), 0.94 - 0.82 (m, 27H).

### Experimental Examples

### Experimental Example 1: Preparation of lipid nanoparticles for in vitro study

Firefly Luciferase encoding mRNA (UTR Optimized Luciferase mRNA N1-Methylpseudouridine(m1Ψ))-encapsulated lipid nanoparticles (LNPs) were formulated with a 20:30:40:0.75 molar ratio of an ionizable lipid, phospholipid, cholesterol and PEG-lipid conjugate. In this case, Compounds 4, 5, 9, 10, 19, 20, 28, 29, 30, and 40 obtained in the above examples were used as the ionizable lipid, and 1,2-dioleoyl-sn-glycero-3-phosphoethanolamine (DOPE) (Avanti Polar Lipids Inc, Cat: 850275P) was used as the phospholipid. In addition, cholesterol purchased from Sigma-Aldrich was used, and 1,2-dimyristoyl-rac-glycero-3-methoxypolyethylene glycol-2000 (DMG-PEG2000) (NOF America Corporation) was used as the PEG-lipid conjugate.

The mRNAs were specifically prepared by the following method: A DNA template was synthesized by PCR amplification of the corresponding plasmid using a forward primer and reverse primer containing 120T at the 5'-end. The DNA template was purified by the QIAquick PCR Purification Kit (Qiagen) and examined by agarose gel electrophoresis. All mRNAs were synthesized by 100% substituted UTP by N-1-methyl-pseudouridine-5'-triphosphate (TriLink) using the AmpliScribe T7-Flash Transcription Kit (Lucigen) for *in vivo* studies, and *in vitro* transcription with wild-type unmodified UTP purified by RNA Clean & Concentrator (Zymo) according to the manufacturer's instructions for *in vitro* studies. The mRNA cap-1 structure was synthesized using the Vaccinia Capping System (NEB) and Cap 2'-O-methyl transferase (NEB), and then another purification was performed by RNA Clean & Concentrator (Zymo). The concentration was measured with a NanoDrop 2000 Spectrophotometer (Thermo), and then all mRNAs were diluted with 1 × TE to the desired concentration, aliquoted, and stored at -80°C for future use. Synthesized mRNAs were analyzed for quality with the TapeStation RNA BioAnalyzer.

The concentration of mRNAs was fixed at 0.01 mg/mL and prepared in a final volume of 100 µL. The mass ratio of ionizable lipid to mRNA was fixed at 10. All lipid substances were dissolved in ethanol at the desired concentration, and mRNAs were dissolved in 10 mM citrate buffer (pH 3.0). Of the final volume of 100 µL, 50 µL was 1X PBS, 25 µL was the aqueous phase, and 25 µL was the ethanol phase. *In vitro* mRNA-encapsulated LNPs were prepared by rapid pipetting.

Cells were previously plated at a density of 20,000 cells/100 µL/well in a white 96-well plate (Corning 3917) overnight. For *in vitro* studies, 5 µL of freshly prepared mRNA-encapsulated LNPs were treated in at least three wells within 1 hour after preparation. 18 hours after treatment, cells were treated with 100 µL of Bright-Glo Luciferase substrate (Promega E2650). After shaking incubation for 5 minutes in the dark, the luminescence intensity was measured with Cytation 5 (Biotek, US). Particle size was measured using a Malvern Zetasizer NanoZS (Malvern, UK).

Additionally, unless otherwise listed, all chemicals and solvents were purchased from Sigma-Aldrich (St. Louis, MO, USA). DOPE (Avanti Polar Lipids Inc, Cat: 850275P). DSPC (Avanti Polar Lipid Inc, Cat: 850365). DMG-PEG2000 (NOF America Corporation). Hep3B, which is a human liver epithelial cell line, was purchased from ATCC (HB-8064). It was cultured in Eagle's Minimum Essential Medium (EMEM) containing 10% FBS. C2C12, which is a mouse myoblast cell line, was purchased from ATCC (CRL-1772). It was cultured in Dulbecco's Modified Eagle's Medium (DMEM) containing 10% FBS. All cells were maintained in a humidified 37°C incubator under 5% CO₂.

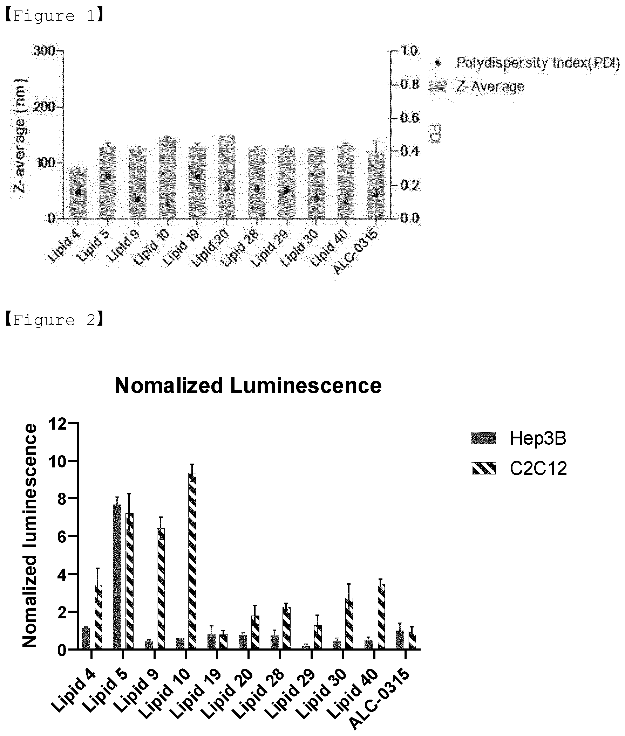

The results of particle size (Z-average and polydispersity index (PDI)) measured according to the above-mentioned descriptions are shown in Figure 1, and the results of luminescence intensity (normalized luminescence) are shown in Figure 2. In Figures 1 and 2, "Lipid N" refers to a lipid nanoparticle comprising Compound N as an ionizable lipid. In addition, for comparison, Figures 1 and 2 also present results for ALC-0315 LNP (BioNTech's Corminaty^{™}), which is a known lipid nanoparticle.

According to Figure 1, lipid nanoparticles prepared with the compound according to one embodiment of the present invention as an ionizable lipid showed a Z-average of 200 nm or less and a PDI of 0.3 or less, and thus have an appropriate level of particle size as lipid nanoparticles.

According to Figure 2, lipid nanoparticles prepared with the compound according to one embodiment of the present invention as an ionizable lipid showed transfection efficiency similar to ALC-0315 in both Hep3B and C2C12 cell lines. The study has demonstrated the feasibility of delivery of lipid nanoparticles to liver and muscle cells.

### Experimental Example 2: Formulation of lipid nanoparticles for in vivo administration

Lipid nanoparticles for *in vivo* evaluation were prepared by a Precision NanoSystems Nanoassemblr microfluidic device (Vancouver, BC, Canada). Unless otherwise specified, lipids were dissolved in ethanol. The mRNAs were diluted in 10 mM citrate buffer (pH 3.0), and then mixed with 1X PBS (pH 7.4). The total flow rate was fixed at 12 mL/min, and the ratio of ethanol phase to aqueous phase was 1:3. For *in vivo* studies, prior to administration, mRNA-encapsulated LNPs were dialyzed in 1X PBS (pH 7.4 buffer) for 90 min using Slide-A-Lyzer Dialysis Cassettes (MWCO 3.5K) purchased from Life Technologies (Grand Island, NY, USA). The total volume change after dialysis (if any) was recorded, and the injection volume was adjusted appropriately for the target dose of mRNAs. The prepared lipid nanoparticles were stored at 4°C for up to 18 hours before administration. The particle size of lipid nanoparticles was measured using a Malvern Zetasizer NanoZS (Malvern, UK) as in Experimental Example 1.

All animal tests were performed in accordance with the Guide for the Care and Use of Laboratory Animals and were approved by the IACUC of The Ohio State University (Approval ID: 2014A00000106-R2) . Wild-type C57BL/6 female mice aged 8-10 weeks were used in all experiments. Three mice were used per group. For intramuscular injections, injections were made in both the left and right hind flanks per mouse. LNPs were injected through intramuscular injection into the back flank of the mouse or intravenous injection into the tail vein. For intramuscular injections, the target dose was 0.8 µg per injection for a dose of 0.04 mg/kg, for a 20 g mouse. LNPs were formulated at an mRNA concentration of 0.02 mg/mL. For intravenous injection, the target dose was 6 µg mRNA per tail vein injection for a dose of 0.3 mg/kg. LNPs were formulated at an mRNA concentration of 0.03 mg/mL. Approximately 6 hours, 28 hours and 54 hours after injection, the luminescence intensity (total flux) in the liver area of the mouse was measured using IVIS Lumina II (Perkin Elmer, Inc.).

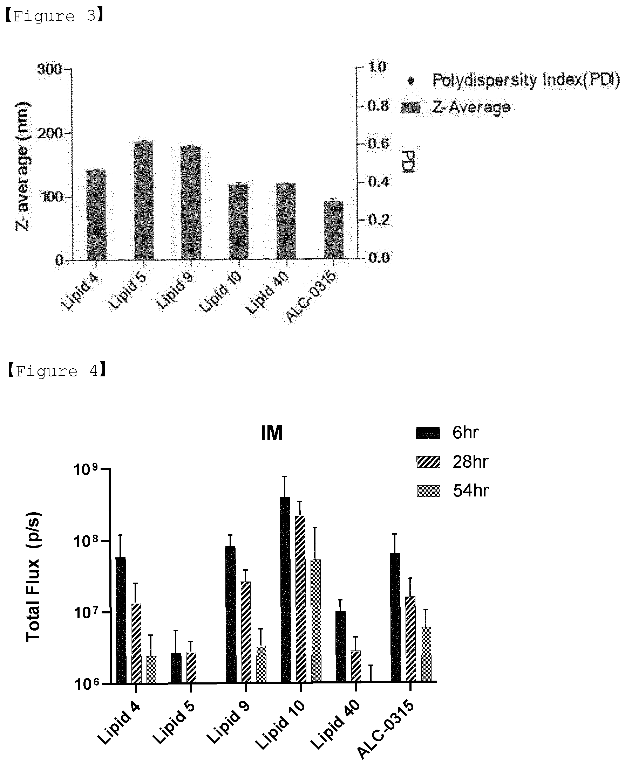

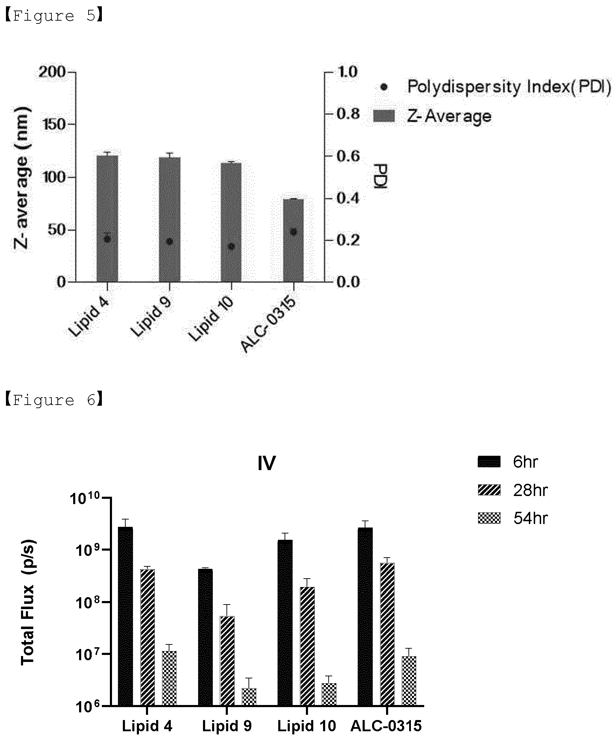

Descriptions not specifically specified in Experimental Example 2 were conducted under the same conditions as Experimental Example 1. Experiments for intramuscular injection (IM) and intravenous (IV) injection were conducted by individually preparing lipid nanoparticles. Lipid nanoparticles comprising Compounds 4, 5, 9, 10 and 40 were prepared as lipid nanoparticles for intramuscular injection, and lipid nanoparticles comprising Compounds 4, 9 and 10 were prepared as lipid nanoparticles for intravenous injection. The results of particle size (Z-average and polydispersity index (PDI)) measured for lipid nanoparticles for intramuscular injection according to the above-mentioned descriptions are shown in Figure 3, and the results of luminescence intensity (total flux) are shown in Figure 4. In addition, the results of particle size (Z-average and polydispersity index (PDI)) measured for lipid nanoparticles for intravenous injection according to the above-mentioned descriptions are shown in Figure 5, and the results of luminescence intensity (total flux) are shown in Figure 6.

According to Figures 3 and 5, the lipid nanoparticles prepared with the compound according to one embodiment of the present invention as an ionizable lipid showed a Z-average of 200 nm or less and a PDI of 0.3 or less when prepared using microfluidic equipment for *in vivo* administration, and thus have an appropriate level of particle size as lipid nanoparticles.

According to Figures 4 and 6, the lipid nanoparticles prepared with the compound according to one embodiment of the present invention as an ionizable lipid were found to efficiently deliver mRNA to liver and muscle *in vivo* through intramuscular injection or intravenous injection, respectively.

Although the present invention has been described focusing on the specific embodiments described above, it should be understood that various modifications and changes to the present invention may be made by those skilled in the art and these also fall within the scope of the present invention as defined by the claims appended below.

## Claims

1. A cycloalkane-based lipid compound of Formula 1 below and a pharmaceutically acceptable salt thereof: 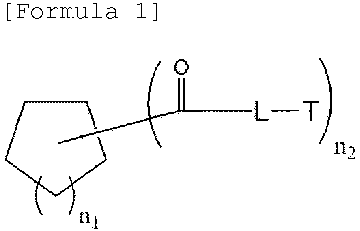 wherein:
n₁ is 1 to 4;
n₂ is 1 to 4;
when n₂ is 2 or 3, each of the substituents linked to a cycloalkane is independent;
L is 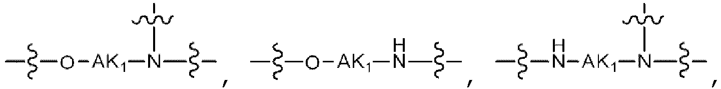 or 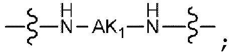
AK₁ is alkylene having 1 to 5 carbon atoms;
T is alkyl or alkenyl having 6 to 20 carbon atoms, or 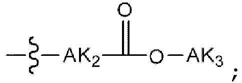
AK₂ is alkylene having 6 to 10 carbon atoms; and
AK₃ is alkyl or alkenyl having 6 to 10 carbon atoms.

2. The cycloalkane-based lipid compound and pharmaceutically acceptable salt thereof according to claim 1, wherein when n₂ is 2 or 3, the substituents linked to the cycloalkane are spaced apart so that one or more carbon atoms are located therebetween.

3. The cycloalkane-based lipid compound and pharmaceutically acceptable salt thereof according to claim 1, wherein n₁ is 2.

4. The cycloalkane-based lipid compound and pharmaceutically acceptable salt thereof according to claim 1, wherein n₂ is 3.

5. The cycloalkane-based lipid compound and pharmaceutically acceptable salt thereof according to claim 1, wherein AK₁ is alkylene having 3 to 5 carbon atoms.

6. The cycloalkane-based lipid compound and pharmaceutically acceptable salt thereof according to claim 1, wherein T is alkyl having 8 to 16 carbon atoms.

7. The cycloalkane-based lipid compound and pharmaceutically acceptable salt thereof according to claim 1, wherein AK₂ is alkylene having 8 to 10 carbon atoms, and AK₃ is alkyl or alkenyl having 8 to 10 carbon atoms.

8. The cycloalkane-based lipid compound or a pharmaceutically acceptable salt thereof according to claim 1, wherein the cycloalkane-based lipid compounds are Compounds 1 to 40 below:

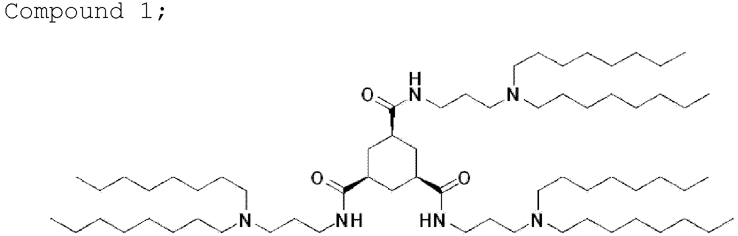

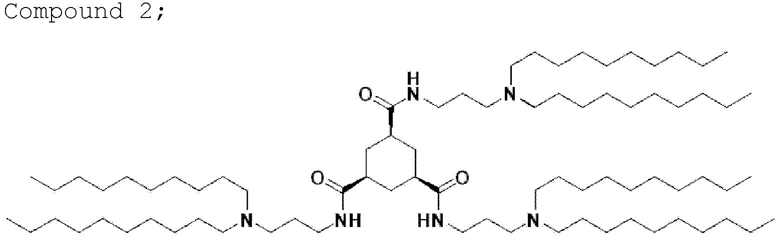

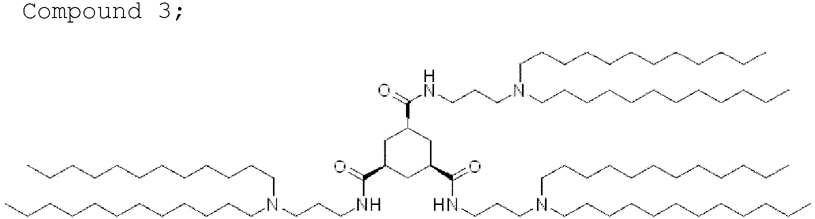

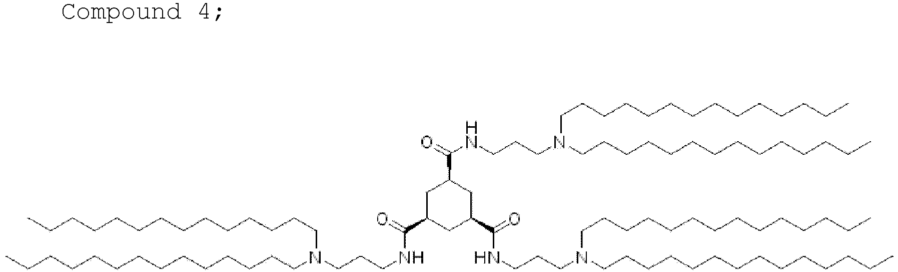

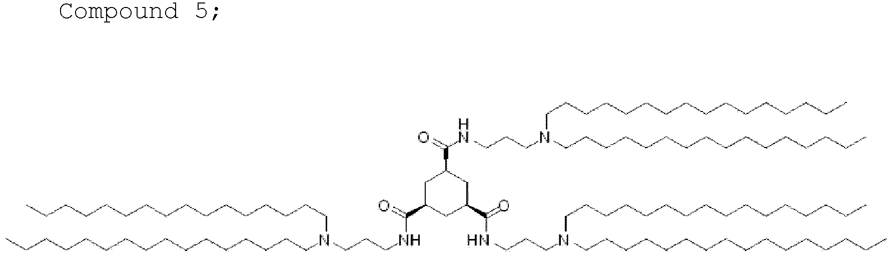

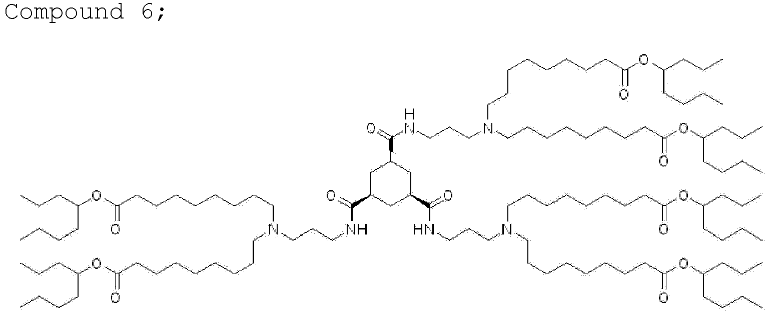

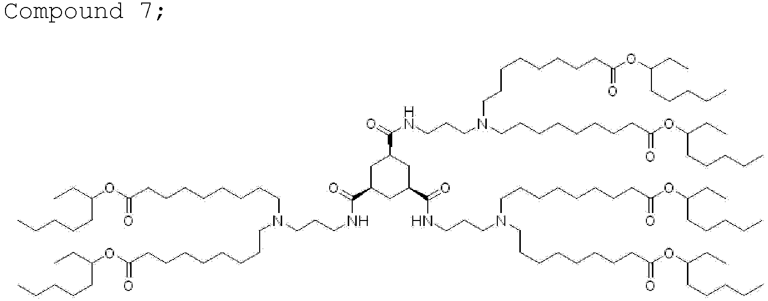

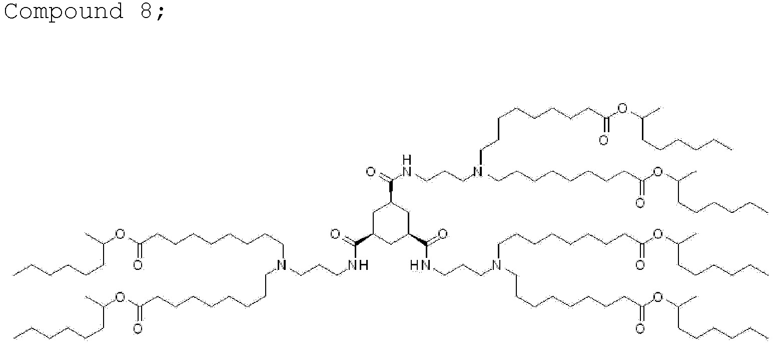

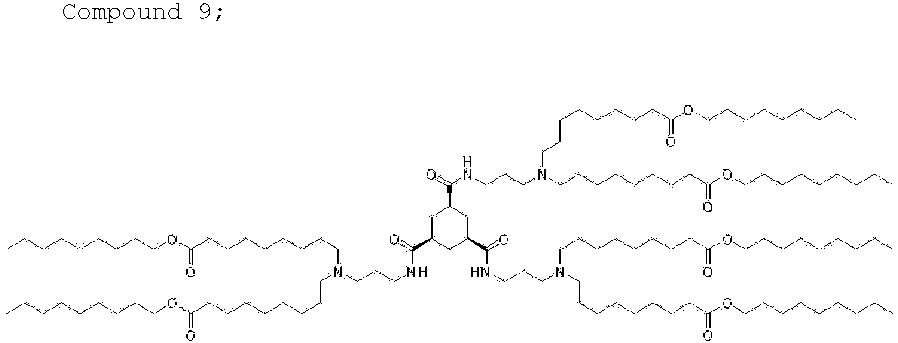

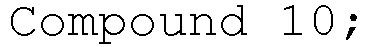

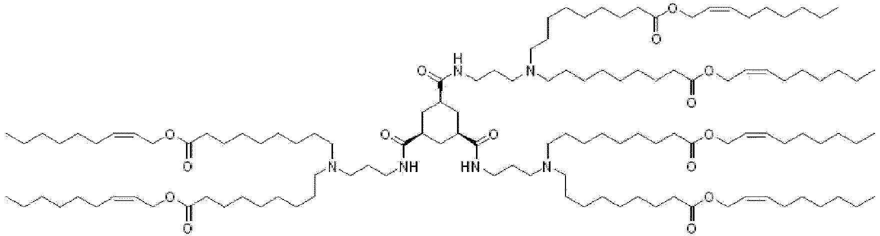

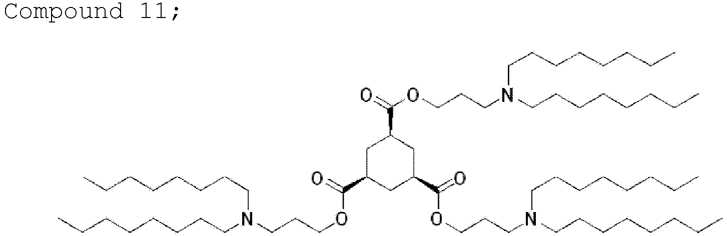

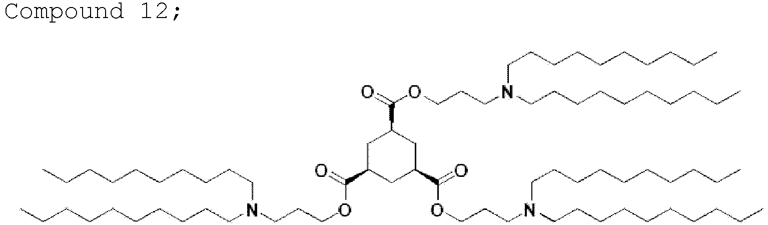

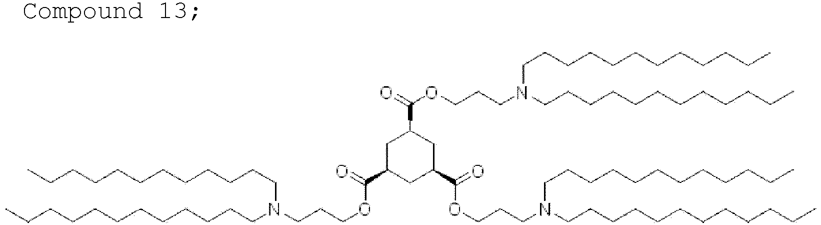

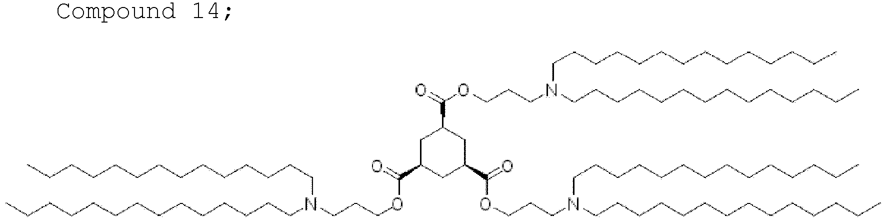

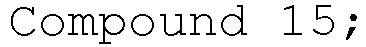

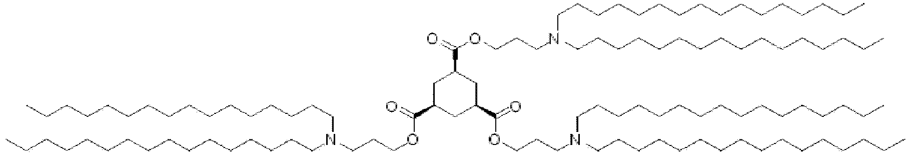

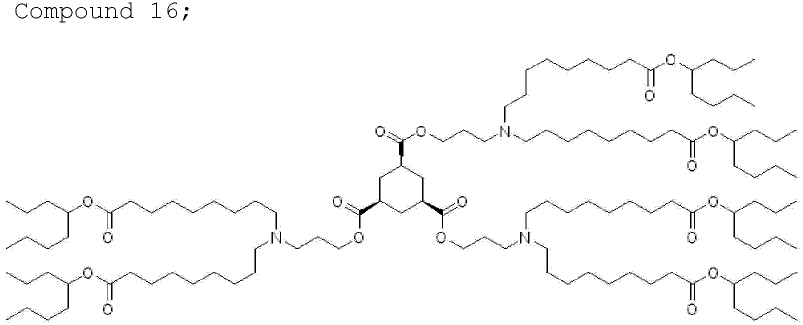

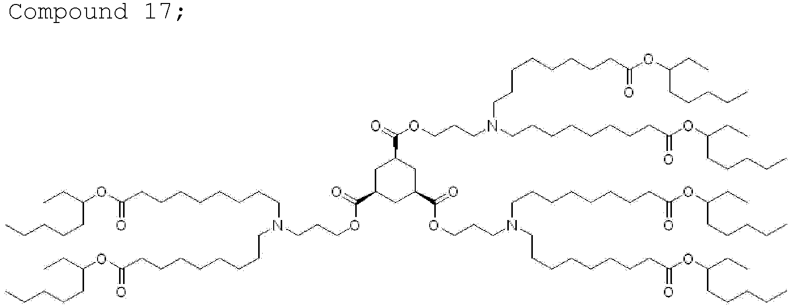

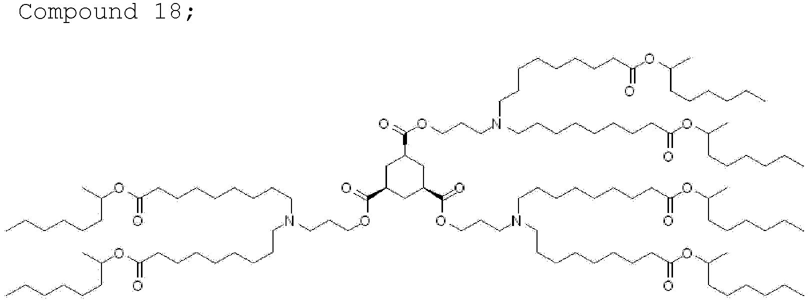

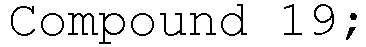

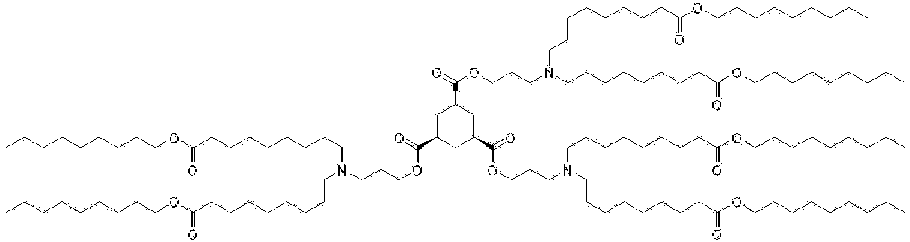

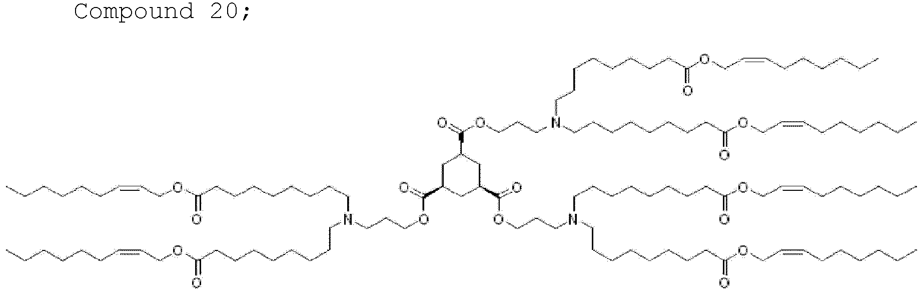

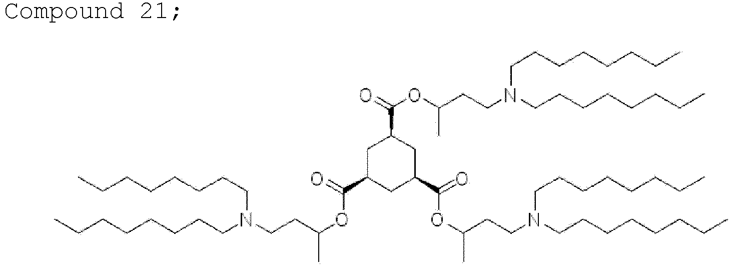

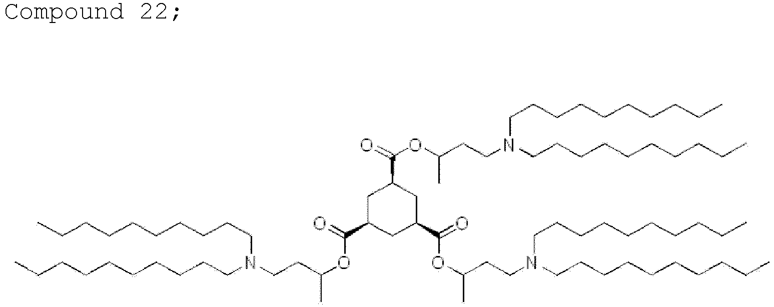

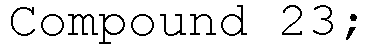

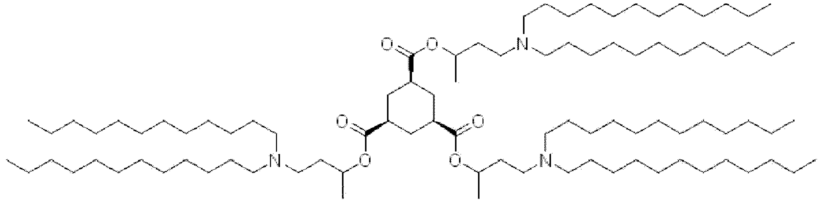

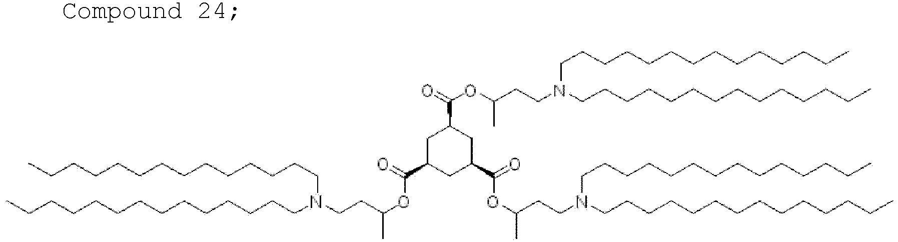

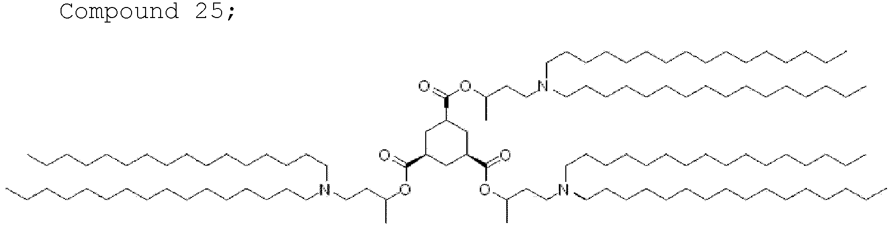

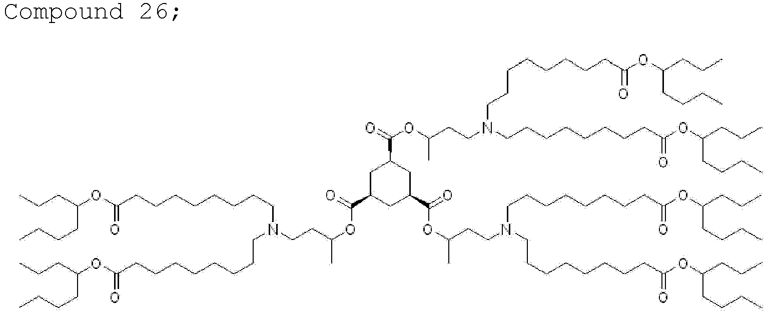

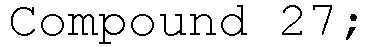

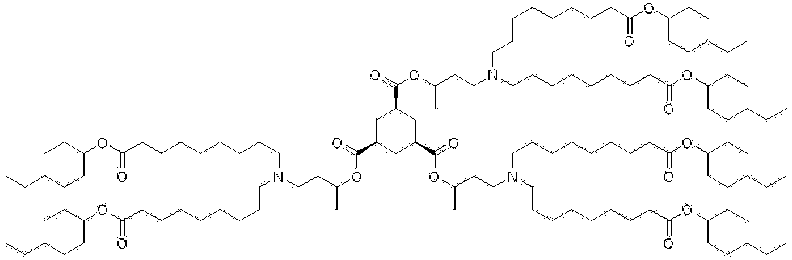

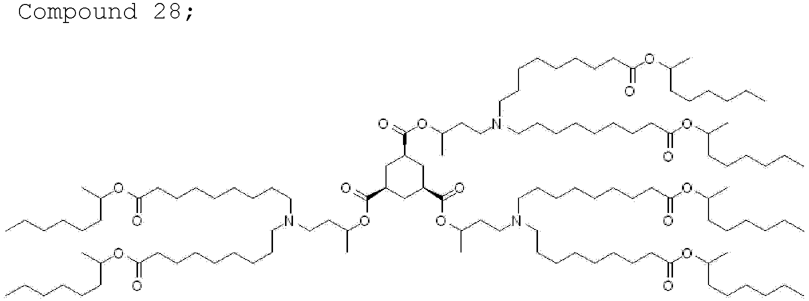

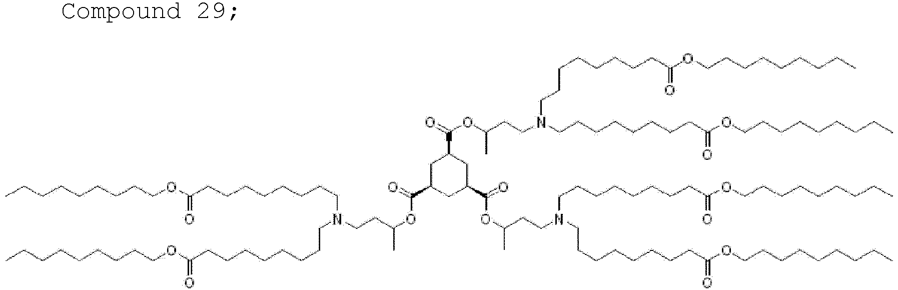

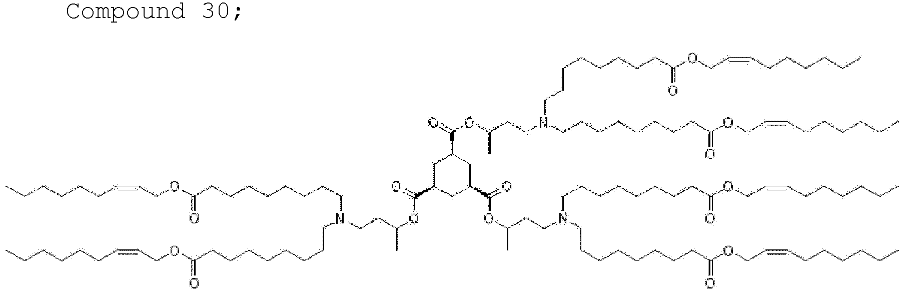

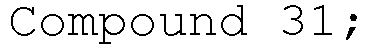

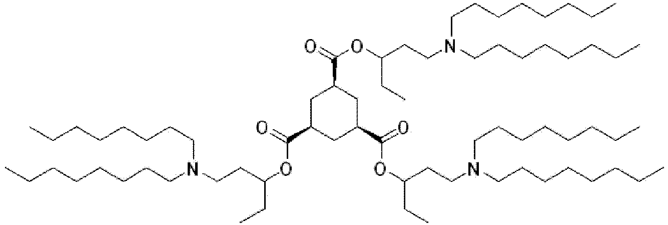

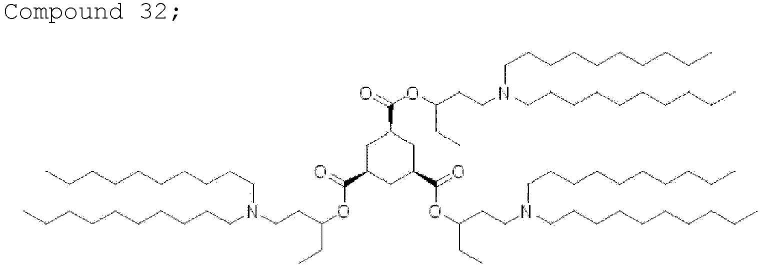

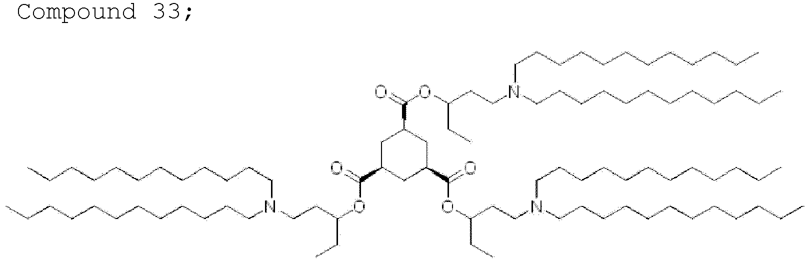

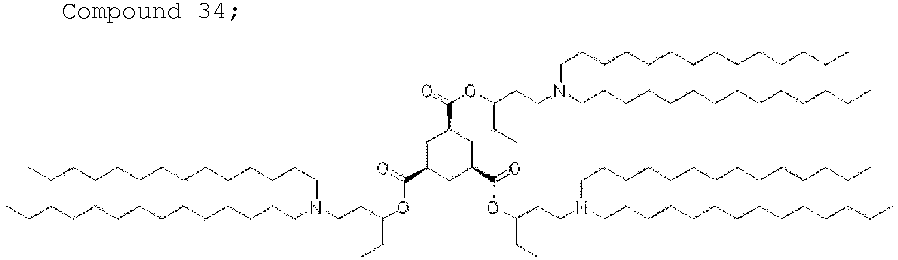

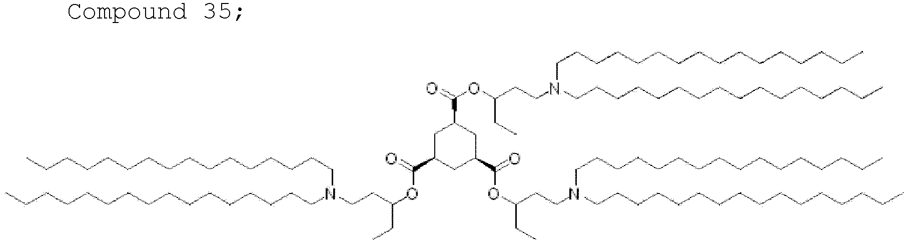

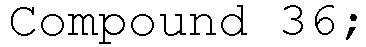

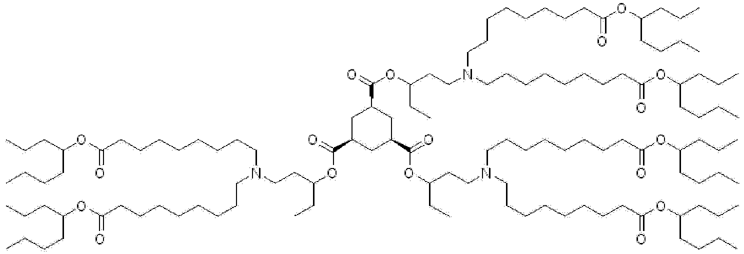

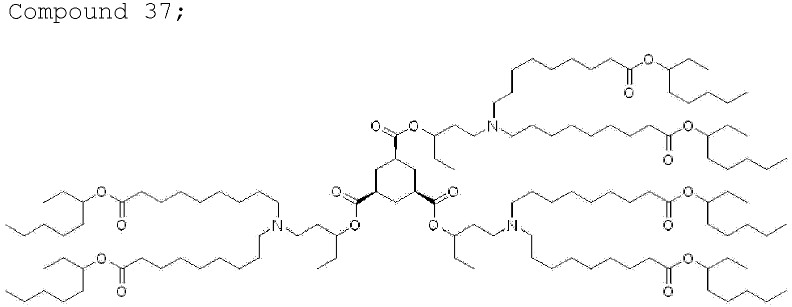

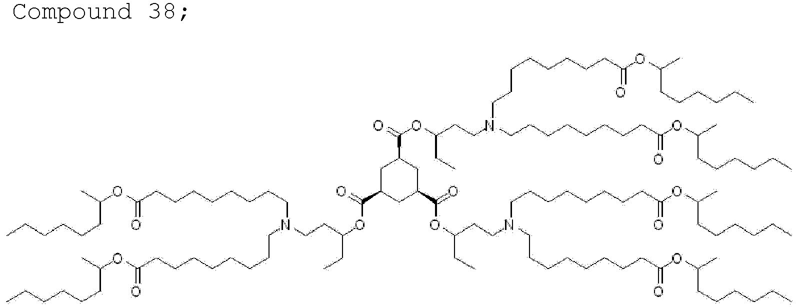

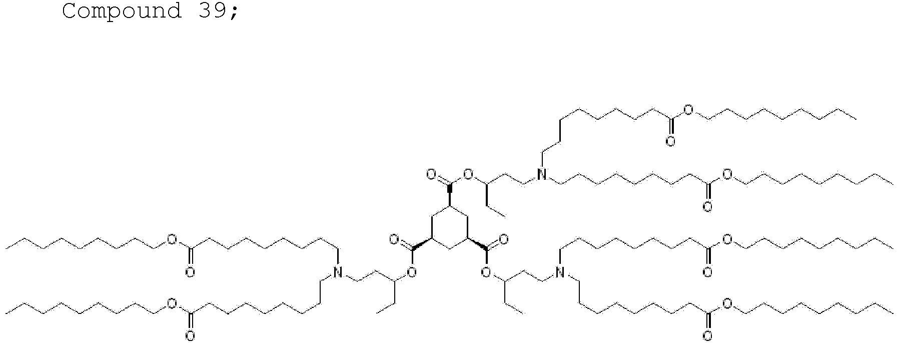

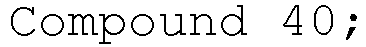

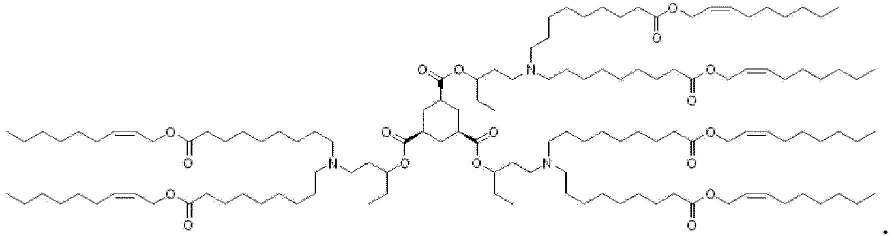

9. A lipid nanoparticle comprising the cycloalkane-based lipid compound according to claim 1, phospholipid, cholesterol and PEG-lipid conjugate.
